# EUROPÄISCHE PATENTSCHRIFT

(11) **EP 3 415 226 B1**
(45) Veröffentlichungstag und Bekanntmachung des Hinweises auf die Patenterteilung: **02.06.2021**
(21) Anmeldenummer: 18176523.1
(22) Anmeldetag: 07.06.2018
(51) Int. Cl.: B01F 13/00, B01F 15/02, A61B 17/88, A61M 5/31

(54) **KNOCHENZEMENTAPPLIKATOR MIT LEITUNGSELEMENT UND VERSCHLUSSAUFNAHME**
BONE CEMENT APPLICATOR WITH CONDUIT ELEMENT AND CLOSURE HOLDER
APPLICATEUR DE CIMENT OSSEUX POURVU D'ÉLÉMENT CONDUCTEUR ET LOGEMENT DE FERMETURE

(30) Priorität: 14.06.2017 DE 102017113126
(43) Veröffentlichungstag der Anmeldung: 19.12.2018
(73) Patentinhaber: Heraeus Medical GmbH, 61273 Wehrheim (DE)
(72) Erfinder: Vogt, Sebastian, 99092 Erfurt (DE); Kluge, Thomas, 56179 Vallendar (DE)
(74) Vertreter: Schultheiss & Sterzel Patentanwälte PartG mbB

(56) Entgegenhaltungen:
- EP-A1- 0 071 288
- WO-A1-00/23002
- WO-A1-2011/089480
- WO-A1-2014/140304
- JP-A- H0 910 281
- US-A- 4 479 801
- US-A1- 2008 195 082
- US-A1- 2011 056 984
- US-A1- 2011 056 985
- US-B1- 6 386 872

## Beschreibung

Die Erfindung betrifft eine Vorrichtung zum Herstellen eines Knochenzementteigs aus einer Monomerflüssigkeit und einem Zementpulver als Ausgangskomponenten des Knochenzementteigs und zum Austragen des gemischten Knochenzementteigs.

Die Erfindung betrifft auch ein Verfahren zur Herstellung eines Knochenzementteigs, insbesondere eines pastenförmigen Polymethylmethacrylat-Knochenzementteigs, mit einer solchen Vorrichtung.

Gegenstand der Erfindung ist insbesondere eine Vorrichtung zum separaten Lagern des Zementpulvers und der Monomerflüssigkeit von Polymethylmethacrylat-Knochenzement, zum anschließenden Vermischen des Zementpulvers mit der Monomerflüssigkeit zur Bildung eines Knochenzementteigs und zum Austragen des gemischten Knochenzementteigs, wobei ein selbsttätiger Verschluss zum Öffnen der Vorrichtung angeordnet ist. Es handelt sich bei der erfindungsgemäßen Vorrichtung bevorzugt um ein Full-Prepacked-Zementiersystem.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). Die Monomerkomponente enthält im Allgemeinen das Monomer Methylmethacrylat und einen darin gelösten Aktivator (N,N-Dimethyl-p-toluidin). Die Pulverkomponente, auch als Zementpulver oder Knochenzementpulver bezeichnet, weist ein oder mehrere Polymere auf, die auf Basis von Methylmethacrylat und Comonomeren, wie Styren, Methylacrylat oder ähnlichen Monomeren durch Polymerisation, vorzugsweise Suspensionspolymerisation, hergestellt sind, einen Röntgenopaker und den Initiator Dibenzoylperoxid auf. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement beziehungsweise Knochenzementteig. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Knochenzementteigs, bis dieser erstarrt.

PMMA-Knochenzemente können in geeigneten Mischbechern mit Hilfe von Spateln durch Vermischen des Zementpulvers mit der Monomerflüssigkeit vermischt werden. Dabei kann es zum Einschluss von Luftblasen im Knochenzementteig kommen, welche die mechanischen Eigenschaften des ausgehärteten Knochenzements negativ beeinflussen können.

Zur Vermeidung von Lufteinschlüssen im Knochenzementteig wurden eine Vielzahl von Vakuum-Zementiersystemen beschrieben, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, EP 1 886 647 A1, US 5 344 232 A.

Aus den Patenten DE 10 2010 019 220 B4, EP 2 596 873 B1 und DE 10 2013 226 118 B3 sowie der Patentanmeldung DE 10 2014 101 305 A1 sind Vorrichtungen zum Mischen von PMMA-Knochenzementen aus zwei pastösen Ausgangskomponenten bekannt.

Eine Weiterentwicklung in der Zementiertechnik stellen Zementiersysteme dar, in denen sowohl das Zementpulver als auch die Monomerflüssigkeit bereits in separaten Kompartimenten der Mischvorrichtungen verpackt sind und die erst unmittelbar vor der Zementapplikation im Zementiersystem miteinander vermischt werden. Solche geschlossenen Full-Prepacked-Mischvorrichtungen wurden mit der EP 0 692 229 A1, der DE 10 2009 031 178 B3, der US 5 997 544 A, der US 6 709 149 B1, der DE 698 12 726 T2, der EP 0 796 653 A2 und der US 5 588 745 A vorgeschlagen.

Das Patent DE 10 2009 031 178 B3 offenbart eine Lager- und Mischvorrichtung als Full-Prepacked-Zementiersystem, in dem die zur Herstellung des Knochenzementteigs notwendigen Ausgangskomponenten bereits in der Lager- und Mischvorrichtung gelagert sind und in der Lager- und Mischvorrichtung zusammengeführt und gemischt werden können. Die Lager- und Mischvorrichtung weist einen zweiteiligen Austragskolben zum Verschluss einer Zementkartusche auf. Dabei wird eine Kombination aus einem gasdurchlässigen Sterilisationskolben und einem gasundurchlässigen Dichtungskolben verwendet.

Polymethylmethacrylat-Knochenzemente werden nach Vermischung des Zementpulvers mit der flüssigen Monomerkomponente im noch nicht ausgehärteten, pastenförmigen Zustand als Knochenzementteig appliziert. Bei Verwendung von Mischvorrichtungen befindet sich der Knochenzementteig im Fall von Pulver-Flüssigkeits-Zementen in einer Kartusche. Bei der Applikation solcher konventioneller PMMA-Knochenzemente, wird nach der Vermischung der beiden Ausgangskomponenten der gebildete Knochenzementteig mit Hilfe von manuell bedienbaren Auspressvorrichtungen ausgepresst. Aus der Kartusche wird der Knochenzementteig durch Bewegung eines Austragskolbens herausgedrückt. Die Austragskolben haben üblicherweise einen Durchmesser zwischen 30 mm bis 40 mm und damit an der Außenseite, an welcher eine Stange (häufig auch als Stößel bezeichnet) der Auspressvorrichtung beim Auspressvorgang angreift, eine Fläche von 7,0 cm² bis 12,5 cm². Die Bewegung des Austragskolbens wird bevorzugt durch manuell bedienbare, mechanische Auspressvorrichtungen bewirkt. Diese manuellen Auspressvorrichtungen erreichen normalerweise eine Auspresskraft im Bereich von ungefähr 1,5 kN bis 3,5 N.

Diese einfachen mechanischen Auspressvorrichtungen nutzen insbesondere Klemmstangen zum Auspressen, die durch einen manuell zu betätigenden Kipphebel angetrieben werden.

Die manuell angetriebenen Auspressvorrichtungen sind seit Jahrzehnten weltweit erprobt und stellen bisher den Stand der Technik dar. Vorteilhaft an diesen Auspressvorrichtungen ist, dass der medizinische Anwender über die aufzubringende Handkraft ein Gefühl für den Eindringwiderstand des Knochenzementteigs in die Knochenstrukturen (Spongiosa) hat.

Bei der Verwendung aller bisher bekannten Full-Prepacked-Zementiersysteme muss der medizinische Anwender mehrere Arbeitsschritte in einer vorbestimmten Reihenfolge an den Vorrichtungen nacheinander durchführen bis der gemischte Knochenzementteig vorliegt und appliziert werden kann. Eine Verwechslung der Arbeitsschritte kann zum Versagen der Mischvorrichtung führen und daher Störungen im OP-Ablauf verursachen. Kostenintensive Schulungen der medizinischen Anwender sind deshalb notwendig, um Anwenderfehler zu vermeiden.

Das Patent US 6 386 872 B1 offenbart eine ähnliche Vorrichtung wie in Anspruch 1 definiert und ein ähnliches Verfahren wie in Anspruch 14 definiert. Es offenbart eine Kapsel enthaltend ein Dentalrestaurationsmaterial, bei dem ein vorspringender Zapfen zum Öffnen eines enthaltenen Monomerflüssigkeitsbehälters und der Kapsel selbst verwendet wird. In der WO 00/35506 A1 wird eine Vorrichtung vorgeschlagen, bei der Polymethylmethacrylat-Zementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpulverpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Konventionelle Zementpulver zeigen zudem das Phänomen, dass auf Grund der unterschiedlichen Oberflächenenergien die Zementpulverpartikel durch Methylmethacrylat nur schlecht benetzt werden. Dadurch dringt das Methylmethacrylat nur relativ langsam in das Zementpulver ein. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzementteigs. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Aus dem Kleb- und Dichtstoffbereich sind auch elektrisch angetriebene Auspressvorrichtungen bekannt. Diese Vorrichtungen können sowohl mit Akkumulatoren und mit Batterien als auch mit Hilfe einer stationären Stromversorgung angetrieben werden. Diese Vorrichtungen können mit ihren zum Teil sehr großen Auspresskräften besonders zähe pastenförmige Massen auspressen. Nachteilig ist jedoch bei der Verwendung von Elektromotoren, dass diese Buntmetalle enthalten und kostenintensiv in der Beschaffung sind. Im steril zu haltenden OP-Bereich müssen derartige Vorrichtungen aufwendig sterilisiert oder sogar ausgetauscht werden. Bei einer elektrischen Verkabelung kann die Bewegung des Anwenders im OP behindert werden.

Weiterhin wurden auch pneumatische Vorrichtungen vorgeschlagen. Diese Geräte erfordern einen stationären oder mobilen Druckluftanschluss (US 2 446 501 A, DE 20 2005 010 206 U1). Dazu sind Druckluftschläuche notwendig, welche die Bewegung des Anwenders behindern können.

Alternativ dazu ist auch die Verwendung von Druckgaspatronen zur Bereitstellung von Druckgas möglich. Dazu wurden Vorrichtungen vorgeschlagen, bei denen der Druckgaszufluss durch ein Ventil und zusätzlich durch ein zweites Ventil der Strom der viskosen Masse gesteuert werden (US 2004/0074927 A1, US 6 935 541 B1). Bei diesen Vorrichtungen sind die Gaspatronen in den Vorrichtungen integriert. Bei derartigen an Druckluft angeschlossenen oder Druckgaspatronen enthaltenden Systemen ist immer eine Druckgasquelle erforderlich, ohne die die Systeme nicht mehr anwendbar sind.

In der nicht vorveröffentlichten DE 10 2016 121 607 A1 wird ein Full-Prepacked-Zementiersystem mit einer Kartusche enthaltend ein Knochenzementpulver vorgeschlagen. In der Kartusche ist ein Austragskolben vorgesehen und hinter der Kartusche eine Monomeraufnahme enthaltend einen Monomerflüssigkeitsbehälter angeordnet. An der Rückseite der Monomeraufnahme befindet sich ein Förderkolben, mit dem der Monomerflüssigkeitsbehälter zerquetscht werden kann und die Monomerflüssigkeit aus der Monomeraufnahme in die Kartusche gepresst werden kann.

Es zeigte sich in praktischen Versuchen, dass der mit dieser Vorrichtung hergestellte Knochenzementteig bei der Verwendung eines geeigneten Zementpulvers und eines geeigneten Gewichtsverhältnisses von Zementpulver zu Monomerflüssigkeit immer eine gute Konsistenz besitzt. Wird der geborstene Monomerflüssigkeitsbehälter beim Monomertransfer maximal komprimiert, dann erhält man reproduzierbar einen guten Zementteig. Der Anwender muss jedoch die Vorrichtung zwischendurch aus der Auspressvorrichtung entfernen, um dioe Kartusche zu öffnen. Anschließend muss die Vorrichtung wieder mit der Auspressvorrichtung verbunden werden. Es wäre für den Anwender vorteilhaft, wenn er die Vorrichtung nur einmal mit der Auspressvorrichtung verbinden müsste.

Ferner kann es bei bestimmten Konstellationen zu einer ungewünschten Veränderung der Konsistenz des Knochenzementteigs am Ende des Auspressvorgangs kommen, bei dem sich das Mischungsverhältnis zwischen dem Zementpulver und der Monomerflüssigkeit verändert hat. Es können jedoch mitunter auch ein bis wenige kleine Monomerblasen im Volumenbereich von wenigen Mikrolitern am Rand des ausgepressten Zementteigs auftreten.

Im Rahmen der vorliegenden Erfindung wurde gefunden, dass dies mit der Wahl und der Stabilität des Monomerflüssigkeitsbehälters sowie mit dem Vordringen der Monomerflüssigkeit zwischen dem Zementpulver und der Innenwand der Kartusche zusammenhängt. Bei unvollständiger Kompression des geborstenen Monomerflüssigkeitsbehälters, die zum Beispiel durch die Wahl eines Monomerflüssigkeitsbehälters mit sehr stabilen Wandungen auftreten kann, kann nämlich ein Rest der Monomerflüssigkeit zwischen dem Austragskolben und dem Förderkolben innerhalb der Fragmente des geborstenen Monomerflüssigkeitsbehälters verbleiben, der am Ende des Auspressens des Knochenzementteigs durch eine anschließende Nachkompression des geborstenen Monomerflüssigkeitsbehälters infolge einer axialen Bewegung des Förderkolbens in Richtung des Austragskolbens durch das Austragsrohr austreten kann. Die Monomerflüssigkeit kann zwischen dem Zementpulver und der Innenwand der Kartusche entlang der Innenwand der Kartusche kriechen, ohne dass sie sich dabei zügig mit umgebenden Zementpulver durchmischt.

Die Aufgabe der vorliegenden Erfindung besteht also darin, die Nachteile des Stands der Technik zu überwinden. Insbesondere besteht die Aufgabe der Erfindung in der Entwicklung einer Vorrichtung zur Lagerung und Vermischung der Ausgangskomponenten von Polymethylmethacrylat-Knochenzement und diese dahingehend zu verbessern, dass der medizinische Anwender die Vorrichtung nur mit der Auspressvorrichtung verbinden muss, anschließend durch wiederholte manuelle Betätigung der Auspressvorrichtung die Monomerflüssigkeit mit dem Zementpulver vermischt wird und durch nachfolgende Betätigung der Auspressvorrichtung der gebildete Knochenzementteig ausgepresst wird, ohne dass zusätzliche Montage- oder Demontageschritte zur Öffnung der Vorrichtung durch den medizinische Anwender notwendig sind. Die Handhabung der Vorrichtung ist dann mit nur einem Montageschritt sehr einfach im Vergleich zu gegenwärtig auf dem Markt befindlichen Zementiersystemen anwendbar. Es ist daher Aufgabe, die in der nicht vorveröffentlichten DE 10 2016 121 607 beschriebene Vorrichtung so zu verbessern, dass die Bildung des Knochenzementteigs reproduzierbar möglich ist, ohne dass die Vorrichtung von der Auspressvorrichtung getrennt und wieder mit ihr verbunden werden muss.

Ferner soll die Vorrichtung zum Mischen des Knochenzementteigs aus den Ausgangskomponenten und zum Austragen des gemischten Knochenzementteigs vorgesehen und geeignet sein. Die Erfindung soll auch ein Verfahrens zur Herstellung eines Knochenzementteigs, insbesondere eines pastenförmigen Polymethylmethacrylat-Knochenzementteigs, bereitstellen, wobei der Knochenzementteig aus einem Zementpulver und einer Monomerflüssigkeit mit einer solchen Vorrichtung hergestellt wird, mit dem die Nachteile der bisherigen Vorrichtungen und Verfahren überwunden werden. Die Erfindung hat insofern auch die Aufgabe die Bildung von Monomerblasen im erzeugten Knochenzementteig zu verhindern. Ferner hat die vorliegende Erfindung die Aufgabe, eine solche Vorrichtung derart zu verbessern, dass auch bei einer nicht vollständigen Kompression des Monomerflüssigkeitsbehälters am Ende des Austrags des Knochenzementteigs ein Austritt der Monomerflüssigkeit aus dem Austragsrohr der Kartusche wirksam verhindert wird. Mit der erfindungsgemäßen Vorrichtung und dem erfindungsgemäßen Verfahren soll also erreicht werden, dass auch bei einem sehr einfachen und kostengünstigen Aufbau der Vorrichtung und bei gleichzeitig sehr einfacher und unkomplizierter Anwendbarkeit der Vorrichtung von Anfang bis Ende des Auspressvorgangs ein homogener Knochenzementteig erzeugt und appliziert werden kann.

Die Vorrichtung soll durch eine einfache Auspressvorrichtung anzutreiben sein und dabei möglichst einfach in der Bedienung sein. Der Aufbau soll kostengünstig sein, damit die Vorrichtung aus hygienischen Gründen nur einmalig verwendet werden kann. Es sollen möglichst viele oder alle in der Vorrichtung ablaufenden Prozesse, wie die Vermischung der Ausgangskomponenten, das Austragen des Knochenzementteigs und gegebenenfalls auch das Öffnen des Monomerflüssigkeitsbehälters und gegebenenfalls auch das Öffnen der Kartusche mit möglichst wenigen Arbeitsschritten und so weit als möglich automatisiert ablaufen und vorzugsweise mit einem einzigen linearen Antrieb anzutreiben sein.

Die Aufgabe der Erfindung besteht also auch in der Entwicklung einer Vorrichtung zum Vermischen von Zementpulver und Monomerflüssigkeit. Die Handhabung der Vorrichtung soll maximal vereinfacht sein, um Anwendungsfehler infolge von fehlerhaft durchgeführten Montageschritten grundsätzlich zu vermeiden. Der medizinische Anwender soll die Vorrichtung nach der Entnahme aus einer Verpackung mit einer Auspressvorrichtung verbinden und diese anschließend betätigen. Weitere Montage- und Arbeitsschritte sollen durch den Aufbau der Vorrichtung vermieden werden. Die Vorrichtung soll vorzugsweise auch eine sichere Lagerung von Zementpulver und Monomerflüssigkeit in voneinander getrennten Kompartimenten ermöglichen, so dass während der Lagerung der Vorrichtung eine unbeabsichtigte Vermischung der Zementkomponenten ausgeschlossen ist. Die Vorrichtung soll eine Sterilisation mit dem Gas Ethylenoxid ermöglichen. Das in der Vorrichtung gelagerte Zementpulver muss dazu für Ethylenoxid zugänglich sein. Die Vorrichtung soll mit Hilfe einer im OP manuell angetriebenen Auspressvorrichtung aktivierbar sein, so dass nach der form- oder kraftschlüssigen Verbindung der Vorrichtung mit der Auspressvorrichtung durch Betätigung der Auspressvorrichtung die axial vortreibbare Stange der Auspressvorrichtung auf die Vorrichtung einwirkt, gegebenenfalls den Monomerflüssigkeitsbehälter öffnet und anschließend bei weiterer Bewegung der Stange die Monomerflüssigkeit in das Zementpulver transferiert. Die Vermischung der Monomerflüssigkeit mit dem Zementpulver soll ohne einen von außen manuell zu bewegenden Mischer erfolgen. Es soll möglichst nur mit der Vorwärtsbewegung der Stange der Auspressvorrichtung die Vermischung der Zementkomponenten unter Bildung des Knochenzementteigs, das Öffnen der Austragsöffnung und das Auspressen des gemischten Knochenzementteigs vorgenommen werden. Bevorzugt soll möglichst nur mit der Vorwärtsbewegung der Stange der Auspressvorrichtung auch das Öffnen des Monomerflüssigkeitsbehälters und der nachfolgende Monomerflüssigkeitstransfer in das Zementpulver vorgenommen werden.

Die Aufgaben der Erfindung werden gelöst durch eine Vorrichtung zum Herstellen eines Knochenzementteigs aus einer Monomerflüssigkeit und einem Zementpulver als Ausgangskomponenten des Knochenzementteigs und zum Austragen des gemischten Knochenzementteigs, die Vorrichtung aufweisend
eine Kartusche mit einem zylindrischen Innenraum, in dem die Ausgangskomponenten gemischt werden, wobei der Innenraum der Kartusche an der Vorderseite bis auf eine Austragsöffnung zum Austreiben des Knochenzementteigs aus dem Innenraum geschlossen ist,
einen Austragskolben, der im Innenraum der Kartusche angeordnet ist und der in Richtung der Austragsöffnung drückbar gelagert ist,
das Zementpulver, das im Innenraum der Kartusche zwischen der Austragsöffnung und dem Austragskolben angeordnet ist,
einen Verschluss, der die Austragsöffnung verschließt und der gegen die Austragsöffnung beweglich gelagert ist, und
ein Leitungselement, das an der Vorderseite der Austragsöffnung angeordnet ist, wobei das Leitungselement eine Verschlussaufnahme zum Aufnehmen zumindest eines Teils des Verschlusses umfasst,
wobei der Verschluss durch einen Druck auf den Knochenzementteig derart in die Verschlussaufnahme hinein drückbar ist, dass die Austragsöffnung geöffnet ist, wobei das Leitungselement mit dem in die Verschlussaufnahme gedrückten Verschluss einen freien Leitungsquerschnitt bereitstellt, durch den der Knochenzementteig aus der Austragsöffnung hindurch und aus der Vorrichtung heraus drückbar ist, wobei
in dem Austragskolben zumindest eine Verbindung von der Rückseite des Austragskolbens zur Vorderseite des Austragskolbens zum Einleiten der Monomerflüssigkeit in den Innenraum der Kartusche vorgesehen ist, wobei die zumindest eine Verbindung für die Monomerflüssigkeit und Gase durchlässig ist und für das Zementpulver undurchlässig ist.

Der Knochenzementteig wird im Rahmen der vorliegenden Erfindung auch dann als Knochenzementteig aufgefasst, wenn die Ausgangskomponenten noch nicht vollständig durchmischt sind, sondern wird bereits dann als Knochenzementteig bezeichnet, wenn die Monomerflüssigkeit und das Zementpulver bereits direkt nach dem Einleiten der Monomerflüssigkeit in das Zementpulver miteinander gemischt sind.

Die Richtungsbezeichnungen in Rahmen der vorliegenden Erfindung werden auf die Flussrichtung des Knochenzementteigs beziehungsweise auf die Applikationsöffnung der Vorrichtung bezogen, wobei die Applikationsöffnung vorne an der Vorrichtung angeordnet beziehungsweise definiert ist. Der Austragskolben wird also von hinten angetrieben und nach vorne in Richtung der Austragsöffnung bewegt und dabei der Knochenzementteig in Richtung der Vorderseite durch die Austragsöffnung, durch den freien Leitungsquerschnitt des Leitungselements hindurchgedrückt (beziehungsweise gepresst) und aus der Applikationsöffnung herausgedrückt (beziehungsweise gepresst).

Der Innenraum der Kartusche hat eine zylindrische Geometrie. Die zylindrische Form ist die einfachste, mit der sich der Innenraum der Kartusche realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche. Die Innenwand des Innenraums der Kartusche kann also durch den Zylindermantel eines Zylinders mit beliebiger Grundfläche realisiert sein, insbesondere mit unterschiedlicher Grundfläche realisiert sein, das heißt auch mit nicht kreisförmigen oder nicht runden Grundflächen. Erfindungsgemäß wird jedoch eine zylindrische Geometrie mit rotationssymmetrischer und insbesondere kreisrunder Grundfläche für den Innenraum (und auch für den Verschluss) bevorzugt, da diese am einfachsten zu fertigen ist.

Die Kartusche, der Austragskolben, das Leitungselement und der Verschluss sind vorzugsweise aus einem thermoplastischen Kunststoff gefertigt, insbesondere mit einem Spritzgussverfahren.

Es kann mit der vorliegenden Erfindung auch vorgesehen sein, dass der Knochenzementteig den Verschluss in der Verschlussaufnahme umfließt, wenn der Knochenzementteig durch das Leitungselement fließt, vorzugsweise der Knochenzementteig entlang wenigstens einer Seitenfläche oder Mantelfläche des Verschlusses an dem Verschluss vorbeifließt.

Dass der Knochenzementteig den Verschluss in der Verschlussaufnahme umfließt, bedeutet, dass der Knochenzementteig in Längsrichtung des Verschlusses an dem Verschluss vorbeifließt.

Hierdurch wird erreicht, dass der Aufbau sehr einfach gehalten werden kann, da keine zusätzlichen Kanäle bereitgestellt werden müssen, durch die der Knochenzementteig den Verschluss im Leitungsmittel umfließt. Zudem wird der Knochenzementteig in die Bewegungsrichtung des Verschlusses gedrückt, so dass die Kraft, die mit dem Knochenzementteig übertragen wird und die zur Bereitstellung des Flusses des Knochenzementteigs genutzt wird, nicht umgeleitet werden muss, wodurch die notwendige Kraft zum Öffnen der Vorrichtung und zum Austreiben des Knochenzementteigs gering gehalten werden kann.

Ferner vorgesehen sein, dass der freie Leitungsquerschnitt auf einer Seite zumindest bereichsweise durch den Verschluss begrenzt ist, vorzugsweise durch eine Seitenfläche oder eine Mantelfläche des Verschlusses begrenzt ist.

Auch hierdurch wird erreicht, dass der Knochenzementteig in die Bewegungsrichtung des Verschlusses gedrückt werden kann, auch um durch das Leitungselement zu fließen, so dass die Kraft, die mit dem Knochenzementteig übertragen wird und die zur Bereitstellung des Flusses des Knochenzementteigs genutzt wird, nicht umgeleitet werden muss, wodurch die notwendige Kraft zum Öffnen der Vorrichtung und zum Austreiben des Knochenzementteigs gering gehalten werden kann.

Des Weiteren kann auch vorgesehen sein, dass der Verschluss fest in der Verschlussaufnahme steckt, wenn er aus der Austragsöffnung in die Verschlussaufnahme hineingedrückt ist.

Hiermit wird verhindert, dass sich der Verschluss in der Verschlussaufnahme bewegt, wenn er im fließenden Knochenzementteig angeordnet ist. Dadurch wird eine Veränderung des Strömungswiderstands des Knochenzementteigs und eine zeitliche Veränderung des Volumenstroms des Knochenzementteigs verhindert.

Zur Vereinfachung des Aufbaus kann vorgesehen sein, dass der Verschluss zumindest bereichsweise zylindrisch ist, insbesondere vollständig zylindrisch ist, und die Verschlussaufnahme eine hohlzylinderförmige Hülse bildet, wobei vorzugsweise in der Mantelfläche der hohlzylinderförmigen Hülse zumindest ein Kanal vorgesehen, wobei der zumindest eine Kanal den freien Leitungsquerschnitt bereitstellt.

Diese Ausführung ist besonders leicht zu fertigen. Zudem kann der Verschluss in axialer Richtung seiner Zylindergeometrie bewegt werden, so dass die Bewegung besonders leicht geführt werden kann.

Dabei kann vorgesehen sein, dass der Innendurchmesser der hohlzylinderförmigen Hülse größer ist als der Außendurchmesser des Verschlusses, vorzugsweise wenigstens 1 mm größer ist als der Außendurchmesser des Verschlusses, besonders bevorzugt zwischen 1 mm und 10 mm größer ist als der Außendurchmesser des Verschlusses.

Die sich hierdurch ergebenden freien Leitungsquerschnitte sind derart angeordnet beziehungsweise derart groß, dass sie den Fluss des Knochenzementteigs nur wenig behindern.

Ferner kann dabei vorgesehen sein, dass die axiale Länge des Innenraums der hohlzylinderförmigen Hülse größer ist als die axiale Länge des Verschlusses, vorzugsweise wenigstens 1 mm größer ist als die axiale Länge des Verschlusses, besonders bevorzugt zwischen 1 mm und 20 mm größer ist als die axiale Länge des Verschlusses.

Dadurch kann der Verschluss in der Hülse versenkt werden und der Knochenzementteig den Verschluss leicht umfließen.

Gemäß einer bevorzugten Weiterbildung der vorliegenden Erfindung kann vorgesehen sein, dass in der Verschlussaufnahme Abstandhalter zur Beabstandung des Verschlusses von der Innenwand der Verschlussaufnahme vorgesehen sind, wobei vorzugsweise die Abstandhalten Leisten sind, die besonders bevorzugt in der Bewegungsrichtung des Verschlusses ausgerichtet sind und/oder die in Flussrichtung des Knochenzementteigs ausgerichtet sind.

Hierdurch wird erreichet, dass der freie Leitungsquerschnitt durch Beabstandung des Verschlusses von der Innenwand der Verschlussaufnahme erreicht wird, wenn der Verschluss in die Verschlussaufnahme gedrückt ist.

Dazu kann auch vorzugsweise vorgesehen sein, dass die Abstandhalter eine Höhe aufweisen, die zumindest einem Drittel des Querschnitts des Verschlusses entsprechen, vorzugsweise zumindest der Hälfte des Querschnitts des Verschlusses entsprechen. Hierdurch werden ausreichend große freie Leitungsquerschnitte erzielt.

Des Weiteren kann es auch vorgesehen sein, dass der freie Leitungsquerschnitt zumindest halb groß ist wie der Querschnitt der Austragsöffnung, vorzugsweise zumindest so groß ist wie der Querschnitt der Austragsöffnung.

Hierdurch wird erreicht, dass der Strömungswiderstand für den Knochenzementteig nicht durch einen zu geringen freien Leitungsquerschnitt des Leitungselements beeinträchtigt wird und gleichzeitig der Aufbau der Vorrichtung kompakt ist.

Es wird mit der vorliegenden Erfindung vorgeschlagen, dass die Länge des Verschlusses in seiner Bewegungsrichtung größer ist, als die Breite in den Richtungen senkrecht dazu.

Die Bewegungsrichtung des Verschlusses entspricht vorzugsweise der Längsrichtung senkrecht zur Austragsöffnung. Dadurch kann die Gefahr reduziert werden, dass der Verschluss bei einer Bewegung in die Verschlussaufnahme hinein verkanntet.

Bevorzugt kann auch vorgesehen sein, dass in der Verschlussaufnahme an der von der Austragsöffnung abgewandten vorderen Stirnwand ein Anschlag zur Begrenzung der Bewegung des Verschlusses angeordnet ist, wobei der Anschlag den Verschluss im vollständig eingedrücktem Zustand von der Stirnwand an der Vorderseite der Verschlussaufnahme beabstandet, so dass zwischen der Vorderseite des Verschlusses und der vorderen Stirnwand der freie Leitungsquerschnitt verbleibt.

Damit wird erreicht, dass der Knochenzementteig hinter dem Leitungselement in die gleiche Richtung weitergeleitet werden beziehungsweise strömen kann, in die er beim Eindrücken des Verschlusses in die Verschlussaufnahme floss.

Des Weiteren kann mit einer besonders bevorzugten Form der vorliegenden erfindungsgemäßen Vorrichtung, die auch zum Lagern der Monomerflüssigkeit geeignet ist und somit ein Full-Prepacked-System bereitstellt, vorgesehen sein,
Des Weiteren kann mit einer besonders bevorzugten Form der vorliegenden erfindungsgemäßen Vorrichtung, die auch zum Lagern der Monomerflüssigkeit geeignet ist und somit ein Full-Prepacked-System bereitstellt, vorgesehen sein, dass die Vorrichtung eine Monomeraufnahme aufweist, in der die Monomerflüssigkeit, insbesondere ein Monomerflüssigkeitsbehälter enthaltend die Monomerflüssigkeit, enthalten ist, wobei die Rückseite der Kartusche mit der Vorderseite der Monomeraufnahme verbunden ist, vorzugsweise derart verbunden ist, dass der Innenraum der Kartusche mit dem Innenraum der Monomeraufnahme fluchtet.

Hierdurch ist die Vorrichtung auch zum Lagern der Monomerflüssigkeit geeignet sowie zum Mischen der Monomerflüssigkeit mit dem Zementpulver innerhalb der Vorrichtung. Die Vorrichtung ist damit ein Full-Prepacked-Zementiersystem. Durch die fluchtenden Innenräume der Kartusche und der Monomeraufnahme kann sichergestellt werden, dass zunächst der Förderkolben durch einen auf die Rückseite des Förderkolbens wirkenden Druck bewegt werden kann und anschließend der Förderkolben zum Antreiben des Austragskolbens genutzt werden kann, indem der Förderkolben gemeinsam mit dem Austragskolben weiter in Richtung der Austragsöffnung gedrückt wird.

Die Monomeraufnahme ist vorzugsweise aus einem thermoplastischen Kunststoff gefertigt, insbesondere mit einem Spritzgussverfahren. Hierdurch kann die Vorrichtung kostengünstig als hygienisches Wegwerfprodukt gefertigt werden.

Bei erfindungsgemäßen Vorrichtungen, bei denen die Monomerflüssigkeit in einem Monomerflüssigkeitsbehälter innerhalb der Vorrichtung angeordnet ist, kann vorgesehen sein, dass der Monomerflüssigkeitsbehälter eine Glasampulle, eine Plastikampulle, ein Kunststofffolienbeutel oder ein Aluminium-Kunststoff-Verbund-Beutel ist. In derartigen Monomerflüssigkeitsbehältern kann die Monomerflüssigkeit besonders lange gelagert werden.

Bei erfindungsgemäßen Vorrichtungen mit Monomeraufnahme ist vorgesehen, dass ein Innenraum der Monomeraufnahme und der Innenraum der Kartusche über eine für die Monomerflüssigkeit und für Gase durchlässige aber für das Zementpulver undurchlässige Verbindung miteinander verbunden sind.

Hiermit wird gestellt, dass das Zementpulver nicht durch die Verbindung in den Innenraum der Monomeraufnahme vordringt, dort vorzeitig mit der Monomerflüssigkeit reagiert und anschließend den Monomertransfer in den Innenraum der Kartusche verhindert. Die Verbindung ist im Austragskolben angeordnet.

Des Weiteren kann vorgesehen sein, dass die Monomeraufnahme einen zylindrischen Innenraum aufweist, in dem die Monomerflüssigkeit, insbesondere ein Monomerflüssigkeitsbehälter enthaltend die Monomerflüssigkeit, angeordnet ist.

Der Innenraum der Monomeraufnahme hat eine zylindrische Geometrie. Die zylindrische Form ist auch hier die einfachste, mit der sich der Innenraum der Aufnahme realisieren lässt. Unter einer zylindrischen Form ist geometrisch die Form eines allgemeinen Zylinders mit einer beliebigen Grundfläche zu verstehen, also nicht nur ein Zylinder mit einer kreisförmigen Grundfläche.

Bevorzugt kann ferner vorgesehen sein, dass in der Monomeraufnahme ein in Längsrichtung der Monomeraufnahme beweglicher Förderkolben angeordnet ist, der von einer Rückseite der Monomeraufnahme aus in Richtung der Vorderseite vortreibbar ist, wobei die Monomerflüssigkeit, insbesondere ein Monomerflüssigkeitsbehälter enthaltend die Monomerflüssigkeit, zwischen dem Förderkolben und dem Austragskolben angeordnet ist. Hiermit wird ein Full-Prepacked-Zementiersystem bereitgestellt, in dem alle Ausgangskomponenten des Knochenzementteigs, nämlich die Monomerflüssigkeit und das Zementpulver enthalten sind und dort auch gelagert werden können.

Der Förderkolben schließt die Monomeraufnahme an deren Rückseite flüssigkeitsdicht ab, bis auf gegebenenfalls vorhandene Belüftungsöffnungen (siehe unten).

Es kann dabei vorgesehen sein, dass an der Vorderseite des Förderkolbens zumindest eine vortretende Spitze, Kante und/oder Schneide zum Brechen des Monomerflüssigkeitsbehälters angeordnet ist.

Durch die Anwendung einer definierten Kraft an einer vorherbestimmten und räumlich begrenzten Stelle kann der Druck an dieser Stelle bei gleicher Kraft erhöht werden und so ein definiertes Brechen des Monomerflüssigkeitsbehälters erreicht werden. Dadurch wird der Ablauf des Aufbrechens des Monomerflüssigkeitsbehälters reproduzierbarer.

Bei erfindungsgemäßen Vorrichtungen mit Förderkolben kann alternativ oder zusätzlich vorgesehen sein, dass der Monomerflüssigkeitsbehälter im Inneren der Monomeraufnahme durch eine Bewegung des Förderkolbens in Richtung der Vorderseite der Monomeraufnahme zu öffnen ist, bevorzugt aufzubrechen oder aufzureißen ist.

Hierdurch wird erreicht, dass durch die axiale lineare Bewegung des Förderkolbens der Monomerflüssigkeitsbehälter geöffnet werden kann. Es kann dadurch eine Auspressvorrichtung mit nur einer Stange als axialer linearen Antrieb verwendet werden, um sowohl den Monomerflüssigkeitsbehälter zu öffnen als auch die Monomerflüssigkeit in die Kartusche zu pressen und auch den Knochenzementteig aus der Kartusche zu pressen.

Es kann auch vorgesehen sein, dass in der Wandung der Monomeraufnahme zumindest eine Belüftungsöffnung angeordnet ist, die den Innenraum der Monomeraufnahme mit der Umgebung verbindet.

Hierdurch kann der Innenraum der Monomeraufnahme mit einem sterilisierenden Gas sterilisiert werden.

Dabei kann vorgesehen sein, dass die zumindest eine Belüftungsöffnung derart dicht im Bereich des Förderkolbens angeordnet ist, dass sie durch eine Bewegung des Förderkolbens in Richtung der Vorderseite der Monomeraufnahme verschlossen wird, bevor ein in der Monomeraufnahme angeordneter Monomerflüssigkeitsbehälter, in dem die Monomerflüssigkeit enthalten ist, durch die Bewegung des Förderkolbens geöffnet ist.

Hierdurch kann die Monomerflüssigkeit nicht aus dem Innenraum der Monomeraufnahme austreten, wenn die zumindest eine Belüftungsöffnung von dem sich in Richtung der Vorderseite der Monomeraufnahme bewegenden Förderkolben verschlossen wird, bevor der Monomerflüssigkeitsbehälter durch die Bewegung des Förderkolbens geöffnet wird, also beispielsweise von dem Förderkolben im Innenraum der Monomeraufnahme zerdrückt, zersplittert oder aufgerissen wird.

Bevorzugt kann vorgesehen sein, dass die Monomeraufnahme und die Kartusche einteilig durch einen röhrenförmigen Behälter gebildet sind.

Dieser Aufbau ist der einfachste und am kostengünstigsten zu realisierende Aufbau.

Ferner kann vorgesehen sein, dass an der Rückseite der Vorrichtung, ein Befestigungsmittel zur Befestigung einer Auspressvorrichtung angeordnet ist, mit der der Austragskolben in Richtung der Austragsöffnung drückbar ist.

Hiermit kann die Vorrichtung an einer Auspressvorrichtung mit einer vortreibbaren Stange angeschlossen und befestigt werden.

Es kann vorgesehen sein, dass das Zementpulver an der Vorderseite des Austragskolbens anliegt, insbesondere vollflächig anliegt, wobei vorzugsweise das Zementpulver in den Innenraum der Kartusche eingepresst ist.

Hierdurch wird verhindert, dass in der Kartusche größere Gaseinschlüsse verbleiben, die beim Mischen der Monomerflüssigkeit mit dem Zementpulver zu Gaseinschlüssen im Knochenzementteig führen könnten. Dies kann bei einem dicht geschütteten oder vorzugsweise gepressten Zementpulver nicht passieren, da die Monomerflüssigkeit die Partikel des Zementpulvers gut benetzt und die Oberflächenspannung der Monomerflüssigkeit dann keine oder zumindest keine relevanten Gaseinschlüsse zwischen den Partikeln des Zementpulvers erlaubt.

Gemäß einer Weiterbildung der vorliegenden Erfindung kann vorgesehen sein, dass an der der Austragsöffnung zugewandten Vorderseite des Austragskolbens ein Hohlzylinder angeordnet ist, wobei der Hohlzylinder an seiner der Austragsöffnung zugewandten Vorderseite offen ist und der sich vorzugsweise von der Vorderseite des Austragskolbens zumindest 3 mm in den Innenraum der Kartusche erstreckt.

Mit dem Hohlzylinder an der Vorderseite des Austragskolbens gelingt es, die Monomerflüssigkeit beim Einpressen in das Zementpulver im Innenraum der Kartusche über eine größere Strecke durch das Zementpulver fließen zu lassen beziehungsweise zu leiten, bevor die Monomerflüssigkeit die Innenwand der Kartusche erreicht. Dadurch kann die Ausbildung von Monomerflüssigkeitsblasen beziehungsweise Einschlüssen der Monomerflüssigkeit in dem gebildeten Knochenzementteig vermieden beziehungsweise reduziert werden. So kann ein homogenerer Knochenzementteig erzeugt werden. Ferner wurde gefunden, dass es durch das Zurückhalten eines kleinen Rests des in der Kartusche entstandenen Knochenzementteigs als Mischung des Zementpulvers mit der Monomerflüssigkeit in dem Innenraum der Kartusche gelingt, dass am Ende des Auspressvorgangs kein Knochenzementteig mit einer veränderten Konsistenz ausgetragen wird, da der restliche Knochenzementteig in der Kartusche zurückgehalten wird und die Austragsöffnung verschlossen wird.

Es kann bevorzugt vorgesehen sein, dass der Austragskolben gegen die Innenwand des Innenraums der Kartusche dicht ist oder abgedichtet ist, insbesondere mit zumindest einer umlaufenden Dichtung abgedichtet ist.

Es kann vorgesehen sein, dass die Austragsöffnung in der Vorderseite der Kartusche angeordnet ist.

Der Hohlzylinder ist im Innenraum der Kartusche angeordnet. Bevorzugt ist die Vorderseite des Austragskolbens bis auf den Hohlzylinder eben.

Dass sich der Hohlzylinder von dem Austragskolben in Richtung der Vorderseite der Kartusche und damit im Innenraum der Kartusche erstreckt, bedeutet, dass ein Totvolumen von dem Hohlzylinder im Innenraum der Kartusche begrenzt wird. Dadurch, dass ein Totvolumen im Innenraum der Kartusche verbleibt, kann zwischen der Austragsöffnung und dem Austragskolben ein Volumen verbleiben, das mit einem Knochenzementteig abweichender Zusammensetzung gefüllt ist, wenn der Hohlzylinder gegen die Vorderseite des Innenraums der Kartusche gedrückt ist und der Austragskolben dadurch nicht weiter in Richtung der Austragsöffnung vorgetrieben werden kann.

Bei erfindungsgemäßen Vorrichtungen kann vorgesehen sein, dass der Hohlzylinder an seiner äußeren Mantelfläche maximal 0,5 mm von der Innenwand des Innenraums der Kartusche beabstandet ist, bevorzugt maximal 0,1 mm von der Innenwand des Innenraums der Kartusche beabstandet ist.

Hierdurch wird sichergestellt, dass sich zwischen der Innenwand des Innenraums der Kartusche und der äußeren Mantelfläche des Hohlzylinders kein oder nur wenig Zementpulver befindet, dass für die Monomerflüssigkeit nur schwer zu erreichen ist und das die Bewegung des Austragskolbens behindern würde.

Es kann auch vorgesehen sein, dass der Hohlzylinder zumindest bereichsweise an der Innenwand des Innenraums der Kartusche anliegt, vorzugsweise an seiner äußeren Mantelfläche an der Innenwand des Innenraums der Kartusche anliegt.

Damit wird sichergestellt, dass sich zwischen der Innenwand des Innenraums der Kartusche und der äußeren Mantelfläche des Hohlzylinders kein Zementpulver befindet, dass für die Monomerflüssigkeit nur schwer zu erreichen ist und das die Bewegung des Austragskolbens behindern würde.

Bei erfindungsgemäßen Vorrichtungen mit Hohlzylinder kann vorgesehen sein, dass der Hohlzylinder eine weitere Bewegung des Austragskolbens in Richtung der Vorderseite der Kartusche blockiert, wenn die Vorderseite des Hohlzylinders an der Vorderseite des Innenraums der Kartusche anliegt, so dass der Austragskolben von der Vorderseite des Innenraums der Kartusche beabstandet ist und ein Totvolumen in dem Innenraum der Kartusche verbleibt.

Damit wird erreicht, dass das in dem Hohlzylinder eingeschlossene Totvolumen einen Rest des erzeugten Knochenzementteigs, der schlechter gemischt ist oder der sich aufgrund von in den Innenraum der Kartusche nachströmender Monomerflüssigkeit am Ende des Auspressvorgangs eine sich ändernde Konsistenz aufweist, in der Kartusche zurückhält. Ferner kann vorgesehen sein, dass der Hohlzylinder zumindest einen Schlitz aufweist, vorzugsweise zumindest einen parallel zur Zylinderachse des Hohlzylinders verlaufenden aufweist, besonders bevorzugt zumindest einen von der Vorderseite bis zum Austragskolben reichenden Schlitz aufweist.

Hierdurch kann die Passung des Hohlzylinders an die Innenwand der Kartusche leichter angepasst werden und die Gefahr einer Blockade der Bewegung des Austragskolbens mit dem Hohlzylinder wird reduziert. Der zumindest Schlitz kann alternativ zu einem Verlauf parallel zur Zylinderachse des Hohlzylinders auch Spiralförmig in der Wandung des Hohlzylinders verlaufen.

Des Weiteren kann vorgesehen sein, dass in dem Austragskolben zumindest eine Verbindung von der Rückseite des Austragskolbens zur Vorderseite des Austragskolbens zum Einleiten der Monomerflüssigkeit in den Innenraum der Kartusche vorgesehen ist, wobei die zumindest eine Verbindung für die Monomerflüssigkeit und Gase durchlässig ist und für das Zementpulver undurchlässig ist und wobei die zumindest eine Verbindung aus dem Austragskolben innerhalb des Hohlzylinders oder durch Leitungen in dem Hohlzylinder an der Vorderseite des Hohlzylinders in den Innenraum der Kartusche münden.

Dadurch muss die Monomerflüssigkeit, wenn sie durch die Durchführung und im Inneren des Hohlzylinders in das Zementpulver geleitet wird zunächst durch das Zementpulver im Inneren des Hohlzylinders fließen und kann nicht an der Innenwand der Kartusche am Zementpulver vorbei strömen und so bis zur Austragsöffnung gelangen. Wenn die Monomerflüssigkeit durch die Leitungen im Hohlzylinder in den Innenraum der Kartusche geleitet wird, strömt diese in einem dichter zu Mitte des Innenraums der Kartusche gelegenen Bereich, so dass sich die Monomerflüssigkeit von dort auch in Richtung des Austragskolbens ausbreiten kann und sich dabei besser verteilt. Bevorzugt ist die Einmündung der Leitungen in den Innenraum der Kartusche im Bereich des inneren Mantels des Hohlzylinders gelagert. Dadurch wird sichergestellt, dass die Monomerflüssigkeit nicht auf kürzestem Weg bis zur Innenwand des Innenraums der Kartusche strömen kann. Alle diese Maßnahmen dienen dazu, dass der erzeugte Knochenzementteig und der aus der Vorrichtung ausgetragene Knochenzementteig eine bessere Homogenität aufweist und sich keine oder möglichst wenige Einschlüsse der Monomerflüssigkeit in dem Knochenzementteig bilden.

Mit einer Weiterbildung der vorliegenden Erfindung wird vorgeschlagen, dass in dem von dem Hohlzylinder umschlossenen Teil des Innenraums der Kartusche Zementpulver enthalten ist, insbesondere eingepresst ist.

Hiermit wird klargestellt, dass in dem von Hohlzylinder umschlossenen Teil ein Totvolumen für den Knochenzementteig gebildet werden soll und dass sich darin nichts Anderes befindet.

Bevorzugt kann ferner vorgesehen sein, dass der von dem Hohlzylinder begrenzte Teil des Innenraums der Kartusche zumindest 1 cm³ groß ist, bevorzugt zumindest 3 cm³ groß ist.

Hiermit wird sichergestellt, dass das eingeschlossene Totvolumen ausreichend groß ist, um die am Ende des Mischvorgangs entstehende Restmenge von sich in der Konsistenz ändernden Knochenzementteig aufzunehmen, ohne dass dieser mit der Vorrichtung ausgetragen und appliziert werden kann. Diese Totvolumina sind ausreichend, um nicht vollständig gemischte Anteile des Knochenzementteigs, die im Bereich des Austragskolbens in dem Innenraum der Kartusche entstehen können, im Innenraum der Kartusche zurückzuhalten. Dadurch kann verhindert werden, dass am Ende des Austragsvorgangs schlecht gemischter Knochenzementteig oder ein Knochenzementteig mit sich ändernder Zusammensetzung und damit Konsistenz ausgetragen wird, der nicht verwendbar ist.

Gemäß einer bevorzugten Weiterbildung der vorliegenden Erfindung kann vorgesehen sein, dass der Hohlzylinder sich von der Vorderseite des Austragskolbens zumindest 5 mm in den Innenraum der Kartusche erstreckt, bevorzugt zumindest 7,5 mm in den Innenraum der Kartusche erstreckt, besonders bevorzugt zumindest 10 mm in den Innenraum der Kartusche erstreckt.

Damit wird einerseits das Totvolumen in dem vom Hohlzylinder eingeschlossenen Bereich vergrößert und andererseits der Weg bis zur Grenzfläche zwischen dem Zementpulver und der Innenwand der Kartusche vergrößert, den die Monomerflüssigkeit durch das Zementpulver zurücklegen muss, bevor die Gefahr besteht, dass die Monomerflüssigkeit an dem Zementpulver oder an bereits entstandenem Knochenzementteig vorbei entlang der Innenwand der Kartusche strömen kann.

Ferner kann vorgesehen sein, dass die Wandstärke des Hohlzylinders zumindest 1 mm beträgt, bevorzugt zumindest 1,5 mm beträgt, besonders bevorzugt zumindest 2 mm beträgt.

Auch diese Maßnahme dient dazu den Weg der Monomerflüssigkeit bis zur Innenwand der Kartusche zu verlängern und dadurch eine größere Homogenität des erzeugten Knochenzementteigs zu erreichen. Zudem wird so eine ausreichende Stabilität des vorzugsweise aus Kunststoff bestehenden Hohlzylinders bewirkt, so dass dieser am Ende des Auspressvorgangs nicht verformt oder nicht zu stark verformt wird.

Es kann auch vorgesehen sein, dass der Verschluss an der dem Innenraum der Kartusche zugewandten Rückseite eine Vertiefung aufweist, in der der vorderste Teil des Zementpulvers enthalten ist.

Dadurch wird erreicht, dass der vorderste Teil des Knochenzementteigs, der in der Vertiefung enthalten ist, in dem Verschluss verbleibt und mit diesem nach vorne geschoben wird. Anschließend umfließt der restliche Knochenzementteig den Verschluss mit dem darin enthaltenen Anteil. In diesem vordersten Anteil ist die Monomerflüssigkeit zuletzt angelangt, wenn sie von der Rückseite aus in das Zementpulver eingepresst wird. Der Anteil des Knochenzementteigs in der Vertiefung kann also eine andere Zusammensetzung haben, als der restliche Knochenzementteig. Dadurch kann also ein weniger stark durchmischter Teil des Knochenzementteigs in dem Verschluss zurückgehalten werden.

Der Verschluss bildet vorzugsweise mit dem Austragskolben ein durch axialen Druck auf den Austragskolben in Richtung der Austragsöffnung wirkenden Druck zu öffnendes Verschlusssystem der Kartusche.

Des Weiteren kann vorgesehen sein, dass das Volumen der Verschlussaufnahme ausreichend groß ist, um zumindest einen Teil des Verschlusses aufzunehmen, wobei vorzugsweise die Verschlussaufnahme ausreichend groß ist, um den Verschluss vollständig aufzunehmen und besonders bevorzugt die Verschlussaufnahme ein größeres Volumen aufweist als das Volumen des Verschlusses.

Hiermit kann der sichergestellt werden, dass der Verschluss in seiner verschobenen und dadurch geöffneten Position dem Fluss des Knochenzementteigs nicht im Wege steht und dadurch das Auspressen des Knochenzementteigs behindert.

Die der vorliegenden Erfindung zugrundeliegenden Aufgaben werden auch gelöst durch ein Verfahren zur Herstellung eines Knochenzementteigs, insbesondere eines pastenförmigen Polymethylmethacrylat-Knochenzementteigs, wobei der Knochenzementteig aus einem Zementpulver und einer Monomerflüssigkeit mit einer Vorrichtung nach einem der Ansprüche 1-13 hergestellt wird, mit folgenden nacheinander ablaufenden Schritten:
a) die Monomerflüssigkeit wird durch die Verbindung in den Innenraum der Kartusche gedrückt, so dass sich die Monomerflüssigkeit mit dem Zementpulver mischt und dort den Knochenzementteig bildet,
b) der Knochenzementteig wird mit dem Austragskolben in Richtung der Vorderseite der Kartusche gedrückt,
c) der Verschluss wird durch den von dem Knochenzementteig auf den Verschluss wirkenden Druck in die Verschlussaufnahme gedrückt und dabei die Austragsöffnung geöffnet,
d) der Knochenzementteig fließt durch den freien Leitungsquerschnitt durch das Leitungselement und wird aus der Vorrichtung ausgetragen.

Dabei kann vorgesehen sein, dass die Vorrichtung vor Schritt a) in eine Auspressvorrichtung eingesetzt wird, die Auspressvorrichtung aufweisend eine axial vortreibbare Stange, wobei vorzugsweise der Austragskolben mit der Stange in Richtung der Austragsöffnung der Kartusche vorgetrieben wird.

Hiermit wird kann eine einfache Vorrichtung durch Anwendung einer handelsüblichen Auspressvorrichtung angewendet werden.

Dabei kann wiederum vorgesehen sein, dass nach dem Einsetzen der Vorrichtung in die Auspressvorrichtung ein Förderkolben, der innerhalb einer an der Rückseite der Kartusche angeordneten Monomeraufnahme an der Rückseite der Monomeraufnahme beweglich gelagert ist, mit der Stange in Richtung der Kartusche vorgetrieben wird, wobei durch die Bewegung des Förderkolbens ein Monomerflüssigkeitsbehälter, in dem die Monomerflüssigkeit enthalten ist, geöffnet wird und die Monomerflüssigkeit aus der Monomeraufnahme in die Kartusche gepresst wird, wobei im Innenraum der Kartusche sich das Zementpulver mit der Monomerflüssigkeit zu dem Knochenzementteig mischt.

Hiermit kann die Vorrichtung als Full-Prepacked-Mischsystem verwendet werden.

Es kann auch vorgesehen sein, dass an der der Austragsöffnung zugewandten Vorderseite des Austragskolbens ein Hohlzylinder angeordnet ist, wobei die Monomerflüssigkeit den Hohlzylinder umfließt, bevor sie bis zur Innenwand der Kartusche gelangt und/oder der Austragskolben auf die Vorderseite der Kartusche trifft, wobei mit dem Hohlzylinder eine weitere Bewegung des Austragskolbens in Richtung der Austragsöffnung blockiert wird und eine Restmenge des Knochenzementteigs in dem von dem Hohlzylinder begrenzten Teil des Innenraums der Kartusche verbleibt.

Bei der Anwendung mit Hohlzylinder wird sichergestellt, dass am Ende des Auspressvorgangs ein schlechter gemischter Rest des Knochenzementteigs oder ein Teil des Knochenzementteigs, der eine veränderte Zusammensetzung aufweist, in der Kartusche zurückgehalten wird und nicht zu Applikation verwendet wird.

Schließlich kann auch vorgesehen sein, dass in Schritt a) die Monomerflüssigkeit durch zumindest eine für das Zementpulver undurchlässige aber für Gase und die Monomerflüssigkeit durchlässige Verbindung im Austragskolben in die Kartusche gepresst wird, vorzugsweise durch eine Bewegung eines Förderkolbens, der mit der Stange der Auspressvorrichtung angetrieben wird, in die Kartusche gepresst wird.

Hiermit wird verhindert, dass sich die Monomerflüssigkeit frühzeitig mit dem Zementpulver mischt.

Der Erfindung liegt die überraschende Erkenntnis zugrunde, dass es mit dem in der Verschlussaufnahme versenkbaren Verschluss und dem freien Querschnitt in dem Leitungsquerschnitt gelingt, ein Öffnen der Kartusche innerhalb der Vorrichtung zu ermöglichen, ohne dass der Verschluss entnommen werden muss oder aus der Vorrichtung herausfällt.

Dazu wurde ein möglichst einfaches Verschlusssystem entwickelt, bei dem sich durch Bewegung des gebildeten Knochenzementteigs beziehungsweise durch einen auf den Knochenzementteig ausgeübten linearen Druck selbsttätig der Verschluss der Vorrichtung öffnet. Der Verschluss beziehungsweise der Verschlusskörper fällt dabei nach Öffnung der Vorrichtung nicht von der Vorrichtung ab oder heraus. Das Verschlusssystem ist so beschaffen, dass das Zementpulver sicher verschlossen ist und dass der Verschluss erst öffnet, wenn sich durch Vermischung der Monomerflüssigkeit mit dem Zementpulver pastöser Knochenzementteig gebildet hat. Das Zementpulver leitet nämlich einen auf den Austragskolben ausgeübten Druck nicht auf den Verschluss weiter, da die Pulverpartikel sich gegen die seitlichen Wandungen der Kartusche abstützen, wenn sie nicht mit der Monomerflüssigkeit gemischt sind. Das Verschlusssystem ist dabei so ausgelegt, dass es ein Bestandteil der Vorrichtung ist und dass der Verschluss derart eingebaut ist, dass Manipulationen von außen nur schwer möglich sind.

Die bevorzugte erfindungsgemäße Vorrichtung in Ihrer Weiterbildung als Full-Prepacked-Zementiersystem hat die wesentlichen Vorteile, dass die beiden Ausgangskomponenten des Knochenzementteigs im geschlossenen Zementiersystem gelagert sind und dass die Vermischung der Ausgangskomponenten in der geschlossenen Vorrichtung erfolgt. Das bedeutet, dass die Vorrichtung nicht vom Anwender befüllt werden muss. Der medizinische Anwender hat keinerlei Kontakt mit den einzelnen Ausgangskomponenten der Knochenzemente. Die Geruchsbelästigung ist dadurch nur noch minimal. Ein besonderer Vorteil der Vorrichtung besteht auch darin, dass durch das einfache Vorwärtsbewegen einer Stange einer manuell angetriebenen Auspressvorrichtung die Monomerflüssigkeit in das Zementpulver gepresst wird. Dabei wird die zwischen den Zementpulverpartikeln vorhandene Luft durch die Monomerflüssigkeit verdrängt. Es entsteht ein homogener Knochenzementteig, ohne dass ein manuelles Mischen mit Mischstäben mit Mischflügeln notwendig ist. Das bedeutet, dass das fehlerbehaftete manuelle Mischen nicht mehr notwendig ist. Die Bedienung der Vorrichtung ist maximal vereinfacht. Es handelt sich um ein Ready-to-use-System.

Die Vorteile erfindungsgemäßer Vorrichtungen und Verfahren beruhen grundsätzlich darauf, dass die an sich bekannte lineare Vorwärtsbewegung von Stangen von manuell betriebenen Auspressvorrichtungen so genutzt werden, dass durch kontinuierliche Einwirkung der Kraft der linearen Vorwärtsbewegung der Stange zuerst ein Monomerflüssigkeitsbehälter geöffnet wird, der Monomerflüssigkeitsbehälter anschließend zusammengepresst wird, wodurch die Monomerflüssigkeit aus dem Monomerflüssigkeitsbehälter austritt und in verdichtetes Zementpulver eingepresst wird, wobei die zwischen den Zementpulverpartikeln vorhandene Luft durch die eingepresste Monomerflüssigkeit verdrängt wird und nach Benetzung der Zementpulverpartikel durch die Monomerflüssigkeit ein Knochenzementteig entsteht. Voraussetzung dafür ist die Verwendung eines Zementpulvers, das so eingestellt ist, dass es sehr gut von der Monomerflüssigkeit benetzt wird und diese durch Kapillarwirkung einsaugen kann.

Die Vorrichtung kann als hygienisches Wegwerfprodukt verwendet werden, da sie sehr weitgehend aus Kunststoff gefertigt werden kann und weil alle Teile einschließlich der Innenräume und des Zementpulvers mit Hilfe von Ethylenoxid sterilisierbar sind.

Eine beispielhafte erfindungsgemäße Vorrichtung zum Lagern, Vermischen und Austragen von Polymethylmethacrylat-Knochenzement kann zusätzlich zu den Merkmalen des Anspruchs 1 beispielsweise aufweisen:
a) einen hohlzylinderförmigen Behälter der vorne eine Kartusche und hinten eine Monomeraufnahme bildet, wobei der Behälter ein am rücksetigen Ende angeordneten Element zur Verbindung mit einer Auspressvorrichtung aufweist,
b) einen Kartuschenkopf, der die hohlzylinderförmige Kartusche abschließt, wobei eine Durchführung zur Aufnahme des Austragsrohrs im Kartuschenkopf angeordnet ist, und wobei mindestens eine Durchführung die Außenseite des Kartuschenkopfs mit der Innenseite des Kartuschenkopfs gasdurchlässig verbindet,
c) ein Austragsrohr,
d) ein axial im Kartuschenkopf beweglichen Sterilisationskolben als Verschluss, der gasdurchlässig aber für Pulverpartikel undurchlässig ist,
e) einen Förderkolben, der in der Monomeraufnahme axial beweglich angeordnet ist und der den Kartuschenboden flüssigkeitsundurchlässig verschließt,
f) einen in der Kartusche axial beweglichen Austragskolben, der in der Kartusche zwischen dem Sterilisationskolben und dem Förderkolben angeordnet ist, wobei der Austragskolben mindestens eine flüssigkeitsdurchlässige und für Pulverpartikel undurchlässige Durchführung zwischen den beiden Stirnseiten besitzt, und wobei am Austragskolben an der zum Kartuschenkopf zeigenden Stirnseite ein Hohlzylinder angeordnet ist, dessen äußere Mantelfläche an der Kartuscheninnenwand anliegt, wobei der Hohlzylinder eine Höhe von mindestens 3 mm in axialer Richtung und eine Wandstärke von mindestens 1 mm hat,
g) mindestens einen Monomerflüssigkeitsbehälter in der Monomeraufnahme, der die Monomerflüssigkeit enthält,
i) Zementpulver, das zwischen dem Austragskolben und dem Kartuschenkopf angeordnet ist, wobei
j) zwischen dem Austragsrohr und der Austragsöffnung des Kartuschenkopfs in axialer Richtung eine hohlzylinderförmige Hülse als Verschlussaufnahme angeordnet ist, wobei der Innendurchmesser der Hülse größer ist als der Außendurchmesser des Sterilisationskolbens, wobei die axiale Länge des Innenraums der Hülse kleiner ist als die axiale Länge des Sterilisationskolbens, wobei
k) an der Innenwand der Hülse Abstandhalter angebracht sind, wobei der radiale Abstand von der Längsachse der Kartusche zu den nach innen zeigenden Kanten der Abstandhalter größer, gleich dem Radius des Sterilisationszylinders sind, und wobei
l) durch axiale Bewegung des Knochenzementteigs der Sterilisationskolben aus der proximalen Kartusche in die Hülse gedrückt wird, wobei nach der Aufnahme des Sterilisationskolbens in die Hülse der Knochenzementteig den Sterilisationszylinder zumindest teilweise umfließt und dann durch eine proximale Öffnung der Hülse austritt.

Ein erfindungsgemäßes Verfahren kann beispielsweise mit der beispielhaften Vorrichtung zur Vermischung des Zementpulvers mit der Monomerflüssigkeit unter Bildung von Knochenzementteig mit den folgenden aufeinanderfolgenden Schritten umgesetzt werden:
a) Verbinden der Auspressvorrichtung mit dem Verbindungselement des hohlzylinderförmigen Behälters,
b) Vortreiben der Stange der Auspressvorrichtung,
c) Verschieben des Förderkolbens in Richtung des Kartuschenkopfs,
d) Komprimieren des mindestens einen Monomerflüssigkeitsbehälters zwischen dem Austragskolben und dem Förderkolben,
e) Bersten oder Reißen des Monomerflüssigkeitsbehälters,
f) Zusammenschieben des geborstenen oder gerissenen Monomerflüssigkeitsbehälters und Auspressen der Luft aus dem Innenraum der Monomeraufnahme und der Monomerflüssigkeit mit dem Förderkolben durch die mindestens eine Verbindung des Austragskolbens in das Zementpulver in den Innenraum der Kartusche,
g) weiteres Zusammenschieben des Monomerflüssigkeitsbehälters und Auspressen der Monomerflüssigkeit mit dem Förderkolben durch die flüssigkeitsdurchlässige Verbindung im Austragskolben und Einleitung der Monomerflüssigkeit durch den Hohlzylinder in das Zementpulver in den Innenraum der Kartusche,
h) Ausbreitung der Monomerflüssigkeit in dem Zementpulver unter gleichzeitiger Verdrängung der Luft aus den Zwischenräumen der Zementpulverpartikel,
i) Benetzung der Zementpulverpartikel mit der Monomerflüssigkeit,
j) Entweichen der Luft aus dem Zementpulver durch den gasdurchlässigen Verschluss,
k) Anquellen der Zementpulverpartikel durch die Monomerflüssigkeit und Auslösung der radikalischen Polymerisation der Monomerflüssigkeit durch Reaktion des Beschleunigers mit dem Initiator,
l) Bildung des Knochenzementteigs aus dem Zementpulver und der Monomerflüssigkeit,
m) Verschieben des Sterilisationskolbens durch axiale Druckbeaufschlagung durch den axial in Richtung Kartuschenkopf gepressten Knochenzementteig in die Hülse,
n) Beendigung der proximalen Bewegung des Sterilisationskolbens durch den Anschlag in der Hülse,
o) Umfließen des Sterilisationskolbens mit dem Knochenzementteig infolge der Vorwärtsbewegung des Förderkolbens und des Austragskolbens, und

Im Folgenden werden weitere Ausführungsbeispiele der Erfindung anhand von fünfzehn schematisch dargestellten Figuren erläutert, ohne jedoch dabei die Erfindung zu beschränken. Dabei zeigt:
Figur 1: eine schematische Querschnittansicht einer ersten beispielhaften erfindungsgemäßen Vorrichtung zum Lagern und Mischen einer Monomerflüssigkeit und eines Zementpulvers;
Figur 2: eine schematische Seitenansicht der Vorrichtung nach Figur 1;
Figur 3: vier schematische Querschnittansichten der Vorrichtung nach den Figuren 1 und 2 mit einer angeschlossenen Auspressvorrichtung übereinander zur Verdeutlichung des Ablaufs eines erfindungsgemäßen Verfahrens;
Figur 4: eine schematische Querschnittansicht als Ausschnittvergrößerung durch den vorderen Teil der erfindungsgemäßen Vorrichtung nach den Figuren 1 bis 3 im verschlossenen Zustand;
Figur 5: eine schematische perspektivische Ansicht auf die Teile einer erfindungsgemäßen Vorrichtung mit Applikationsrohr und einer Austragsrohrverlängerung;
Figur 6: vier schematische perspektivische Querschnittansichten des vorderen Teils der Vorrichtung nach den Figuren 1 bis 5 im geschlossenen Zustand und im geöffneten Zustand;
Figur 7: eine schematische Querschnittansicht als Ausschnittvergrößerung der Vorrichtung nach der zweiten Abbildung von oben von Figur 3 beim Einpressen der Monomerflüssigkeit;
Figur 8: eine schematische Querschnittansicht als Ausschnittvergrößerung der Vorrichtung nach der dritten Abbildung von oben von Figur 3 beim nach vorne Pressen des Knochenzementteigs;
Figur 9: vier schematische Querschnittansichten als Ausschnittvergrößerung des vorderen Teils der Vorrichtung im geöffneten und im geschlossenen Zustand;
Figur 10: eine schematische Querschnittansicht einer zweiten beispielhaften erfindungsgemäßen Vorrichtung zum Lagern und Mischen einer Monomerflüssigkeit und eines Zementpulvers als Spine-Applikator zur Spondylodese mit einer angeschlossenen Auspressvorrichtu ng;
Figur 11: eine schematische perspektivische Ansicht des Spine-Applikators nach Figur 10;
Figur 12: vier schematische Querschnittansichten des Spine-Applikators nach den Figuren 10 und 11 mit einer angeschlossenen Auspressvorrichtung übereinander zur Verdeutlichung des Ablaufs eines erfindungsgemäßen Verfahrens;
Figur 13: eine schematische Querschnittansicht als Ausschnittvergrößerung durch den vorderen Teil des erfindungsgemäßen Spine-Applikators nach den Figuren 10 bis 12 im verschlossenen Zustand;
Figur 14: zwei schematische perspektivische Querschnittansichten des vorderen Teils des Spine-Applikators nach den Figuren 10 bis 13 im geschlossenen Zustand und im geöffneten Zustand; und
Figur 15: eine schematische Querschnittansicht als Ausschnittvergrößerung des Spine-Applikators nach der letzten Abbildung von oben von Figur 12.

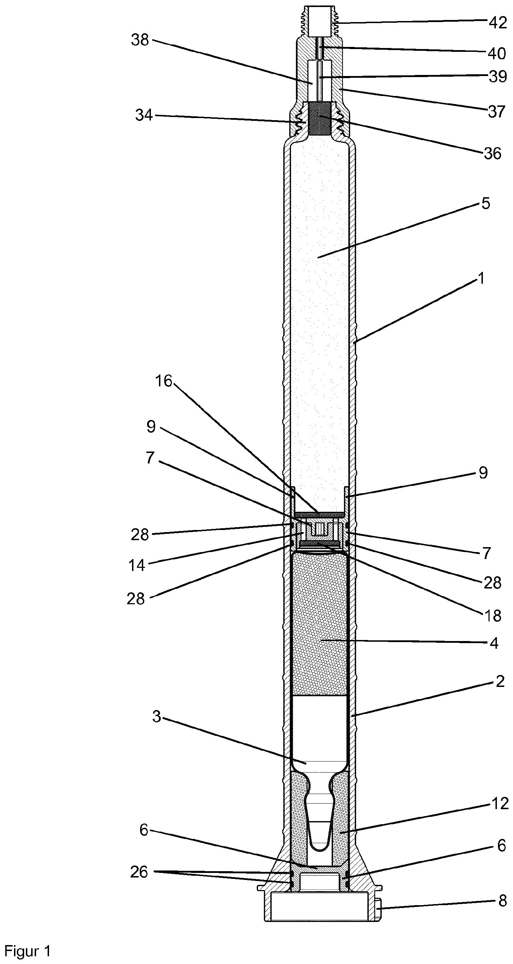

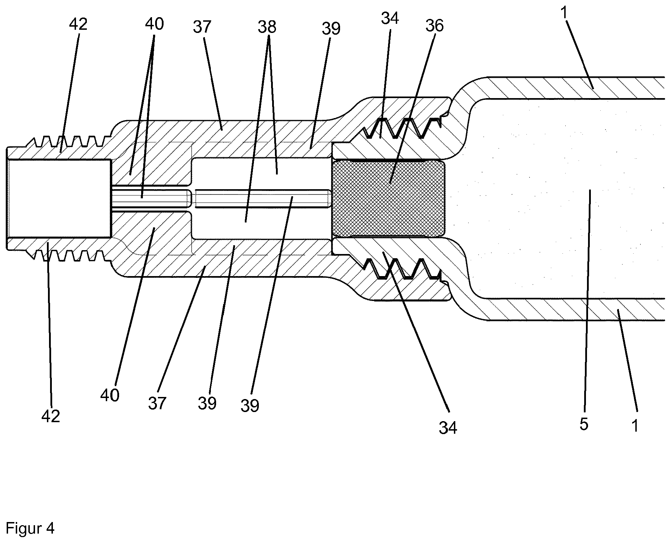

In den Figuren 1 bis 9 sind Abbildungen einer ersten erfindungsgemäßen Vorrichtung gezeigt. Die Figuren 1 bis 3 und 5 zeigen verschiedene schematische Gesamtansichten der beispielhaften erfindungsgemäßen Vorrichtung. Die Figuren 4 und 6 bis 9 zeigen schematische Querschnittansichten als Detailansichten in Form von Ausschnittvergrößerungen durch verschiedene Bereiche der ersten erfindungsgemäßen Vorrichtung.

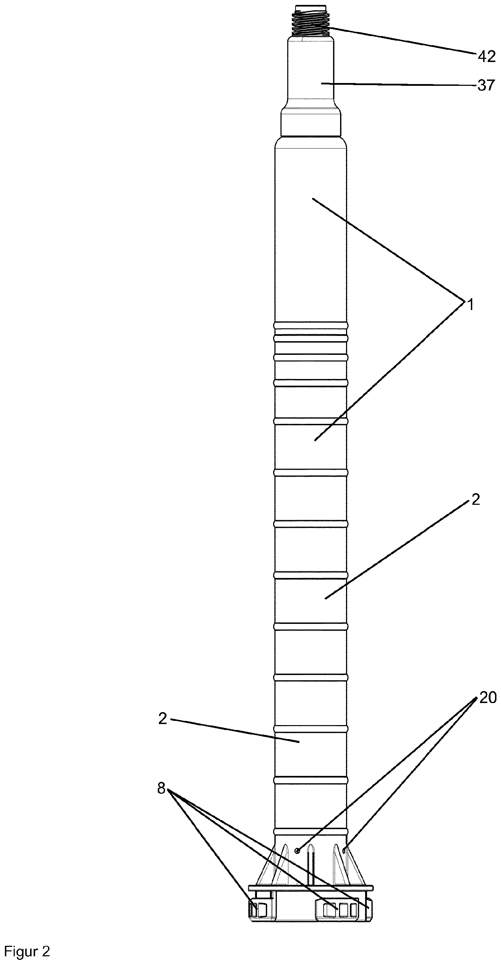

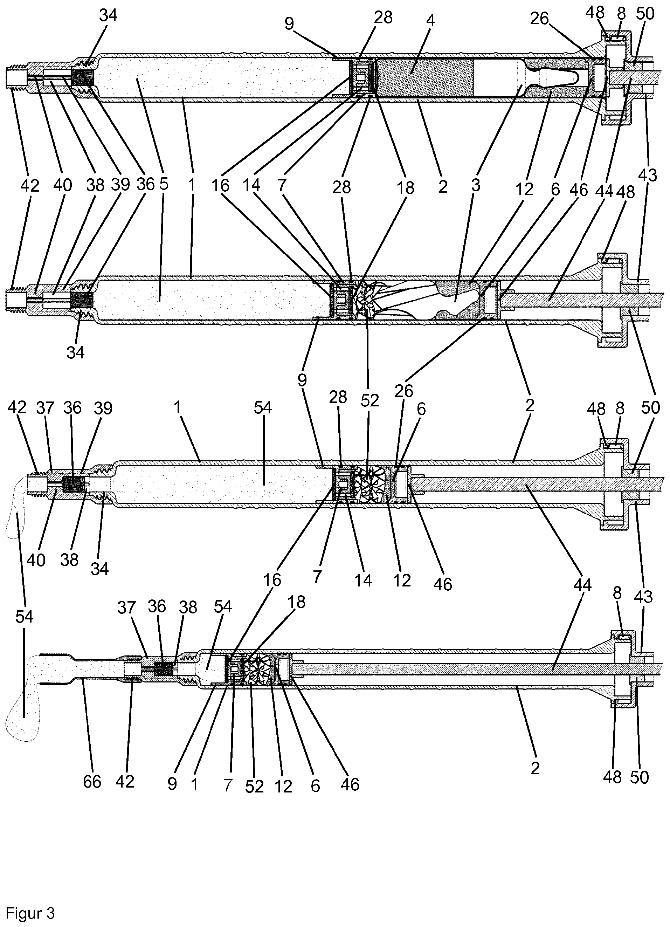

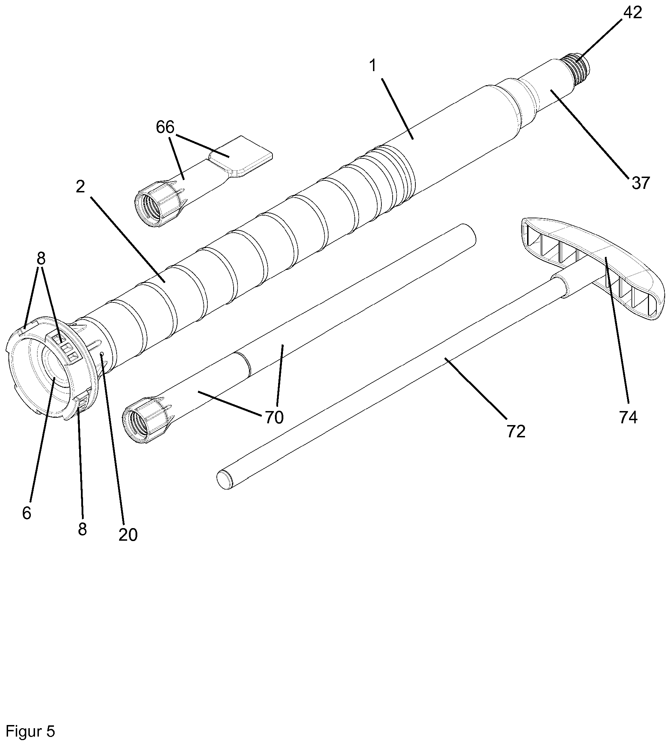

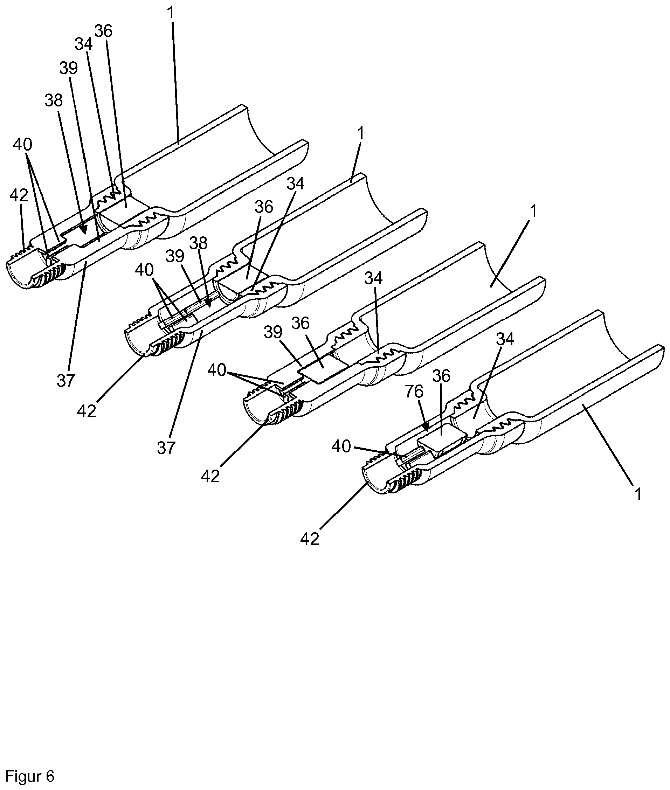

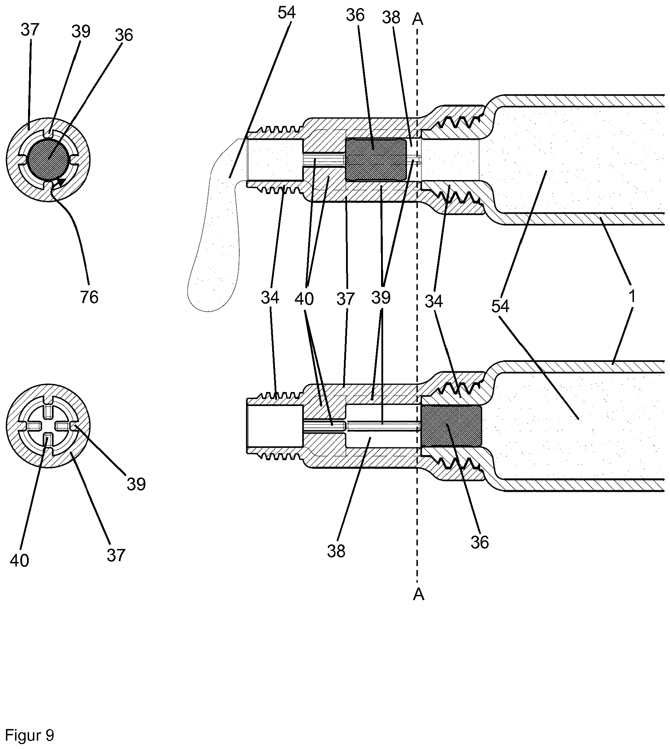

Die erste erfindungsgemäße Vorrichtung besteht im Wesentlichen aus einem röhrenförmigen Behälter aus Kunststoff, der als vorderen Teil (in den Figuren 1 und 2 oben, in den Figuren 3, 4, 7 bis 8 links, in Figur 5 rechts oben, in Figur 6 links unten, in den beiden rechten Abbildungen von Figur 9 links und in den beiden linken Abbildungen von Figur 9 in der Betrachtungsebene) eine Kartusche 1 mit einem zylindrischen Innenraum bildet und der als hinteren Teil eine Monomeraufnahme 2 für eine Glasampulle 3 als Monomerflüssigkeitsbehälter bildet. Anstelle der Glasampulle 3 kann auch ohne weiteres eine aufbrechbare Kunststoffampulle verwendet werden oder es kann durch geringe Umbaumaßnahmen auch ein aufreißbarer Folienbeutel aus einem Metall-beschichteten Kunststoff anstelle der Glasampulle 3 verwendet werden.

Die Rückseite der Vorrichtung ist in den Figuren 1 und 2 unten, in den Abbildungen der Figur 3 rechts und in Figur 5 unten links gezeigt. Die Röhrenform des Behälters ist besonders gut in den Querschnittansichten der Figuren 1 und 3 sowie der perspektivischen Ansicht nach Figur 5 zu erkennen. Sowohl der Innenraum der Kartusche 1 als auch der Innenraum der Monomeraufnahme 2 sind zylindrisch mit kreisförmiger Grundfläche. Dabei sind die Durchmesser des Innenraums der Kartusche 1 und der Durchmesser des Innenraums der Monomeraufnahme 2 gleich groß und fluchten. Der Behälter mit der Monomeraufnahme 2 und der Kartusche 1 wird vorzugsweise mit Hilfe einer Spritzgusstechnik aus Kunststoff hergestellt. Die Monomeraufnahme 2 weist also einen zylindrischen Innenraum auf, in den die Glasampulle 3 eingesteckt ist. In der Glasampulle 3 befindet sich die Monomerflüssigkeit 4. In Figur 1 ist die Vorrichtung umgedreht gezeigt, so dass die Gravitation nach oben wirkt und sich die Monomerflüssigkeit 4 im oberen Teil der Glasampulle 3 sammelt. In den Innenraum der Kartusche 1 ist ein Zementpulver 5 eingefüllt oder vorzugsweise eingepresst. Die Monomerflüssigkeit 4 und das Zementpulver 5 bilden die Ausgangskomponenten für einen PMMA-Knochenzement, der mit der Vorrichtung herstellbar ist. Aufgrund der Glasampulle 3 kann die Monomerflüssigkeit 4 sehr lange in der Monomeraufnahme 2 und dadurch in der Vorrichtung gelagert werden. Das Zementpulver 5 kann ebenfalls über längere Zeiträume in der Vorrichtung gelagert werden. Die Vorrichtung ist damit zum Lagern der Monomerflüssigkeit 4 und des Zementpulvers 5 als Ausgangskomponenten eines Knochenzementteigs des PMMA-Knochenzements geeignet. Die Vorrichtung ist aber auch zum Mischen des Knochenzementteigs aus den Ausgangskomponenten und zum Austragen des gemischten Knochenzementteigs geeignet und vorgesehen.

In der Monomeraufnahme 2 ist ein im zylindrischen Innenraum der Monomeraufnahme 2 in Längsrichtung beweglicher Förderkolben 6 aus Kunststoff angeordnet. Der Förderkolben 6 ist im Bereich der Rückseite der Monomeraufnahme 2 angeordnet. Die Glasampulle 3 kann mit dem Förderkolben 6 in der Monomeraufnahme 2 zusammengedrückt und dabei zersplittert werden, indem der Förderkolben 6 in Richtung der Vorderseite, also in Richtung der Kartusche 1 gedrückt wird. Der Förderkolben 6 weist an der Vorderseite Abstreifer auf, mit denen Splitter der Glasampulle 3 von der Innenwand der Monomeraufnahme 2 abgestreift werden. Die Abstreifer liegen hierzu seitlich an der Innenwand des Innenraums der Monomeraufnahme 2 an.

In dem Innenraum der Kartusche 1 ist in dessen Rückseite (in den Figuren 1 und 2 Richtung unten, in den Figuren 3, 4, 7 und 8 Richtung rechts) ein Austragskolben 7 aus Kunststoff angeordnet. An der Rückseite der Monomeraufnahme 2 ist ein Befestigungsmittel 8 vorgesehen, mit dem die Monomeraufnahme 2 beziehungsweise der Behälter an einer Auspressvorrichtung 43 (in Figur 1 und 2 nicht zu sehen, siehe aber Figur 3) verbunden werden kann. Das Befestigungsmittel 8 ist vorzugsweise zur Bildung eines Bajonettverschlusses 8 geeignet und vorgesehen. Dadurch kann der Förderkolben 6, der von der Rückseite der Monomeraufnahme 2 aus frei zugänglich ist, mit der Auspressvorrichtung 43 in Richtung der Vorderseite der Kartusche 1 vorgetrieben werden.

Der Austragskolben 7 weist an seiner Vorderseite einen Hohlzylinder 9 zum Verlängern der Strecke auf, die die Monomerflüssigkeit 4 durch das Zementpulver 5 fließen muss, bis sie zur Innenwand der Kartusche 1 gelangt. Zudem dient der Hohlzylinder 9 zur Beabstandung des Austragskolbens 7 von einer Austragsöffnung an der Vorderseite des Innenraums der Kartusche 1 sowie zum Schaffen eins Totvolumens zwischen dem Austragskolben 7 und der Vorderseite des Innenraums der Kartusche 1, wenn der Austragskolben 7 beziehungsweise der Hohlzylinder 9 maximal an die Vorderseite des Innenraums der Kartusche 1 gedrückt ist.

Der Hohlzylinder 9 ist vorliegend rotationssymmetrisch und ist nach Art eines Rohrstücks geformt. Der Hohlzylinder 9 kann aber auch parallel zur Zylinderachse des Hohlzylinders 9 verlaufende Längsschnitte aufweisen. An der Vorderseite ist der Hohlzylinder 9 eben.

In dem Innenraum der Monomeraufnahme 2 ist eine Lagerung 12 aus Schaumstoff vorgesehen, die als Transportsicherung und als Stoßsicherung für die Glasampulle 3 dient. Damit soll verhindert werden, dass die Glasampulle 3 bei Erschütterungen oder Stößen ungewollt aufbricht. Der Schaumstoff und damit die Lagerung 12 sind durchlässig für Gase.

Die Kartusche 1 und die Monomeraufnahme 2 sind einteilig als gemeinsames Kunststoffteil ausgeführt. Die Monomeraufnahme 2 und die Kartusche 1 sind für die Monomerflüssigkeit 4 flüssigkeitsdurchlässig miteinander über eine Verbindung 14 im Austragskolben 7 verbunden. Die Verbindung 14 durch den Austragskolben 7 mündet durch einen für das Zementpulver 5 undurchlässigen aber für die Monomerflüssigkeit 4 durchlässigen Porenfilter 16 in den Innenraum der Kartusche 1.

In der Einmündung zur Verbindung 14 ist in dem Austragskolben 7 ein Filter 18 angeordnet, mit dem die Splitter der Glasampulle 3 zurückgehalten werden können. Statt dem Filter 18 oder zusätzlich zum Filter 18 kann auch ein Sieb vorgesehen sein.

Mehrere Belüftungsöffnungen 20 sind in der Wandung der Monomeraufnahme 2 vorgesehen, durch die der Innenraum der Monomeraufnahme 2 mit Hilfe eines sterilisierenden Gases wie Ethylenoxid sterilisiert werden kann. Die Lagerung 12 ist ebenfalls gasdurchlässig und verschließt daher nicht die Belüftungsöffnungen 20. Die Belüftungsöffnungen 20 sind unmittelbar benachbart zum Förderkolben 6 angeordnet, so dass der Förderkolben 6 sich unmittelbar vor die Belüftungsöffnungen 20 schiebt und somit die Belüftungsöffnungen 20 unmittelbar verschließt, wenn der Förderkolben 6 in Richtung der Kartusche 1 vorgetrieben wird. Dadurch wird verhindert, dass Monomerflüssigkeit 4 durch die Belüftungsöffnungen 20 austreten kann, wenn die Glasampulle 3 in der Monomeraufnahme 2 geöffnet wurde.

Der zylindrische Förderkolben 6 hat einen zur Zylindergeometrie des Innenraums der Monomeraufnahme 2 passenden Außenumfang und ist über zwei umlaufende Dichtungen 26 gegen die Innenwand der Monomeraufnahme 2 flüssigkeitsdicht abgedichtet. Ebenso ist der Austragskolben 7 über zwei umlaufende Dichtungen 28 gegen die Innenwand der Kartusche 1 flüssigkeitsdicht abgedichtet. Diese Dichtungen 26, 28 dienen dazu, einen Austritt von Monomerflüssigkeit 4 oder von Knochenzement zu verhindern, um eine Kontamination der Umgebung (des OP-Saals und des Anwenders) zu verhindern. Die Dichtungen 26, 28 können hierzu aus Gummi bestehen.

Der Innenraum der Kartusche 1 mündet an der Vorderseite in einen Stutzen 34, der die Austragsöffnung der Kartusche 1 begrenzt. Der Stutzen 34 weist ein Außengewinde auf. Im Inneren des Stutzens 34 ist ein Verschluss 36 für die Kartusche 1 angeordnet, der in der Austragsöffnung steckt und diese verschließt. Der Verschluss 36 ist ein für das Zementpulver 5 undurchlässiger aber für Gase durchlässiger Porenfilter und hat eine zylindrische Form.

An dem Außengewinde des Stutzens 34 ist ein Leitungselement 37 mit einer Verschlussaufnahme 38 zur Aufnahme des Verschlusses 36 befestigt. Die Verschlussaufnahme 38 ist nach Art einer Hülse geformt und weist vier in Längsrichtung ausgerichtete, sich in die Verschlussaufnahme 38 hinein erhebende Leisten 39 auf. Die Leisten 39 beabstanden den Verschluss 36 von der Innenwand der Verschlussaufnahme 38, wenn der Verschluss 36 in die Verschlussaufnahme 38 hineingedrückt ist. Vor der Verschlussaufnahme 38 verengt sich das Leitungselement 37. In diesem Bereich sind vier weitere Leisten 40 angeordnet, die einen Anschlag 40 für die Bewegung des Verschlusses 36 bilden und also die Bewegung des Verschlusses 36 in die Verschlussaufnahme 38 begrenzen. Zwischen den Leisten 39, 40 ist ein ausreichender freier Leitungsquerschnitt 76 (siehe Figuren 6 und 9) vorgesehen, so dass der Knochenzementteig zwischen den Leisten 39, der Wandung der Verschlussaufnahme 38 und dem eingeschobenen Verschluss 36 sowie zwischen den Leisten 40 im vorderen Teil des Leitungselements 37 hindurch strömen kann. An seiner Vorderseite endet das Leitungselement 37 in einem Stutzen 42 mit einem Außengewinde.

Durch den als Porenfilter ausgeführten Verschluss 36 hindurch können das Innere der Kartusche 1 und das Zementpulver 5 mit Hilfe von Ethylenoxid sterilisiert werden, da das Leitungselement 37 offen ist und der Verschluss 36 und die Zwischenräume zwischen den Pulverpartikeln des Zementpulvers 5 luftdurchlässig sind. Gleichzeitig kann Luft aus der Monomeraufnahme 2 durch das Zementpulver 5, den Verschluss 36 und das offene Leitungselement 37 herausgepresst werden, wenn der Förderkolben 6 in Richtung der Monomeraufnahme 1 gepresst wird.

Das Zementpulver 5 ist in der Kartusche 1 eingeschlossen, da alle Öffnungen und Verbindungen 14 mit Hilfe der Porenfilter 16, 36 für das Zementpulver 5 undurchlässig verschlossen sind. Der Inhalt der Kartusche 1 kann dabei durch Evakuieren und Spülen mit Ethylenoxid sterilisiert werden. Dadurch ist die Vorrichtung auch zum langfristigen Lagern des Zementpulvers 5 geeignet.

In Figur 5 sind neben der Vorrichtung auch ein Applikationsrohr 66 und eine Austragsrohrverlängerung 70 für die Vorrichtung gezeigt, die jeweils alternativ zueinander auf den Stutzen 42 des Leitungselements 37 aufgeschraubt werden können. Dazu weisen das Applikationsrohr 66 und die Austragsrohrverlängerung 70 ein zu dem Außengewinde des Stutzens 42 des Leitungselements 37 passendes Innengewinde auf. Die Austragsrohrverlängerung 70 kann mit einem Verschluss 72 verschlossen sein. Der Verschluss 72 endet in einem Griff 74, mit dem die Austragsrohrverlängerung 70 bequem mit der Hand auf das Leitungselement 37 geschraubt werden kann, wenn der Verschluss 72 in der Austragsrohrverlängerung 70 steckt. Zudem kann mit dem Griff 74 der Verschluss 72, der die der Kartusche 1 zugewandte Seite der Austragsrohrverlängerung 70 verschließt, bequem entfernt werden, auch wenn die Austragsrohrverlängerung 70 mit dem Leitungselement 37 fest verschraubt ist.

Figur 6 zeigt vier schematische perspektivische Querschnittansichten des vorderen Teils der Vorrichtung im geschlossenen Zustand (die beiden Abbildungen auf der linken Seite) und im geöffneten Zustand (die beiden Abbildungen auf der rechten Seite). Die Querschnittebenen liegen dabei in der Längsachse der Vorrichtung und sind dabei gegeneinander um 45° gegeneinander gekippt, so dass bei der ersten Abbildung von oben links und bei der dritten Abbildung von oben links die Leisten 39, 40 in Längsrichtung geschnitten sind und bei der zweiten Abbildung von oben links und bei der vierten Abbildung von oben links der Schnitt zwischen den Leisten 39, 40 gesetzt ist. Dadurch sind bei der Querschnittansicht zwischen den Leisten 39, 40 die Freiräume und insbesondere der sich zwischen den Leisten 39, der Innenwand der Verschlussaufnahme 38 und der Mantelfläche des Verschlusses 36 erhaltene freie Leitungsquerschnitt 76 bei geöffnetem Verschluss 36 (beziehungsweise bei in die Verschlussaufnahme 38 versenktem Verschluss 36) erkennbar. Durch den freien Leitungsquerschnitt 76 und durch die Freiräume zwischen den Leisten 40 kann der Knochenzementteig bei geöffnetem Verschluss 36 strömen.

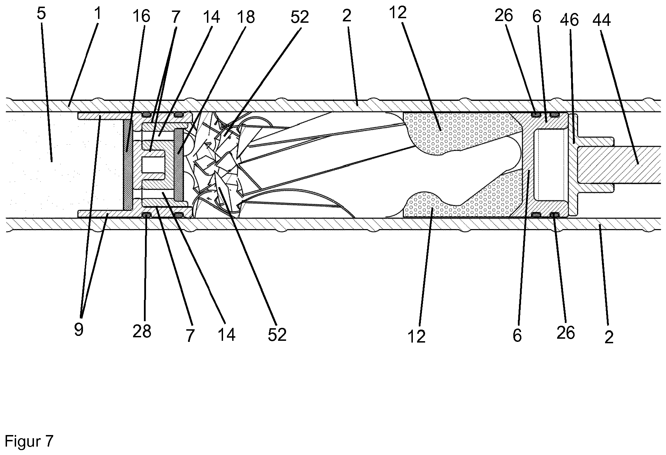

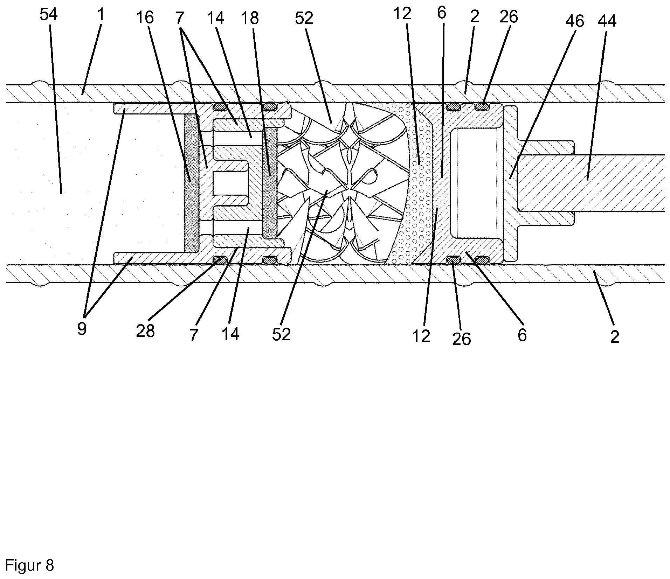

Figur 3 zeigt vier schematische Querschnittansichten der erfindungsgemäßen Vorrichtung übereinander zur Verdeutlichung des Ablaufs eines erfindungsgemäßen Verfahrens. Dazu zeigt Figur 4 eine Ausschnittvergrößerung der ersten Abbildung von oben der Figur 3, Figur 7 eine Ausschnittvergrößerung der zweiten Abbildung von oben der Figur 3 und Figur 8 eine Ausschnittvergrößerung der dritten Abbildung von oben der Figur 3. Die Abbildung in Figur 9 oben rechts zeigt eine Ausschnittvergrößerung der dritten Abbildung von oben der Figur 3 und die Abbildung in Figur 9 unten rechts zeigt eine Ausschnittvergrößerung der zweiten Abbildung von oben der Figur 3. Dazu zeigt die Abbildung in Figur 9 oben links eine senkrechte Querschnittansicht entlang der Schnittebene A-A mit Blickrichtung nach vorne (von der Kartusche 1 weg) und die Abbildung in Figur 9 unten links eine senkrechte Querschnittansicht entlang der Schnittebene A-A mit Blickrichtung nach vorne (von der Kartusche 1 weg).

Zu Beginn des Verfahrens liegt die Vorrichtung im Ausgangszustand vor, so wie sie auch in Figur 1 gezeigt ist. In diesem Zustand wird die Vorrichtung in eine Auspressvorrichtung 43 eingesetzt, beispielsweise eine herkömmliche mit der Hand manuell antreibbare Kartuschenpistole. Diese Situation ist in der obersten Abbildung von Figur 3 gezeigt. Die Auspressvorrichtung 43 umfasst eine linear vortreibbare Stange 44. Von der Auspressvorrichtung 43 ist nur der vordere Teil dargestellt. Die Auspressvorrichtung 43 umfasst auch einen Griff und einen Kipphebel (in den Abbildungen nicht zu sehen) zum manuellen Antreiben der Stange 44 der Auspressvorrichtung 43, wie bei herkömmlichen manuell angetriebenen Auspressvorrichtungen auch. Die Vorrichtung wird mit dem Befestigungsmittel 8 an der Auspressvorrichtung 43 befestigt (siehe oberste Abbildung in Figur 3). An der Spitze der Stange 44 ist ein flacher Teller 46 zum Antreiben des Förderkolbens 6 vorgesehen. Die Stange 44 drückt mit dem Teller 46 auf den Förderkolben 6, wenn die Stange 44 von der Auspressvorrichtung 43 in die Monomeraufnahme 2 hineingedrückt wird. Die Auspressvorrichtung 43 ist hierzu über ein Gegenbefestigungsmittel 48 an die Rückseite der Monomeraufnahme 2 angeschlossen, so dass der Teller 46 beim Vortreiben der Stange 44 auf den Förderkolben 6 drückt und diesen in Richtung der Kartusche 1 vortreibt. Hierzu ist die Stange 44 gegen ein Lager 50 und darüber gegen das Gegenbefestigungsmittel 48 und damit gegen die Monomeraufnahme 2 linear beweglich gelagert.

Die Auspressvorrichtung 43 wird bedient und dabei die Stange 44 und mit der Stange 44 der Förderkolben 6 in Richtung der Kartusche 1 vorgetrieben. Zu Beginn der Bewegung des Förderkolbens 6 verschließt dieser die Belüftungsöffnungen 20. Die Lagerung 12 wird komprimiert und der Förderkolben 6 trifft auf den Kopf der Glasampulle 3. Da die Glasampulle 3 an der Vorderseite an dem Austragskolben 7 anliegt und sich der Innenraum der Monomeraufnahme 2 weiter verkleinert, wird die Glasampulle 3 zerbrochen. Die Monomerflüssigkeit 4 tritt aus der Glasampulle 3 in den Innenraum der Monomeraufnahme 2 aus. Der Austragskolben 7 kann nicht oder nicht weit von der Glasampulle 3 in Richtung des Verschlusses 36 geschoben werden, wenn das Zementpulver 5 trocken ist, also nicht von der Monomerflüssigkeit 4 benetzt ist, da das trockene Zementpulver 5 nicht fließfähig ist und eine Bewegung des Austragskolbens 7 blockiert. Diese Situation ist in Figur 3, zweite Abbildung von oben, sowie in der vergrößerten Ausschnittansicht in Figur 7 gezeigt. Überstehende Luft aus der Monomeraufnahme 2 wird durch den Filter 18, die Verbindung 14, den Porenfilter 16, durch die Zwischenräume zwischen den Partikeln des Zementpulvers 5, durch den Verschluss 36 und aus dem offenen Leitungselement 37 oder aus einem auf das Leitungselement 37 aufgeschraubten Applikatorrohr 66 aus der Vorrichtung herausgedrückt.

Von der Glasampulle 3 bleiben schließlich nur kleine Splitter 52 zurück, die von dem Filter 18 zurückgehalten werden und in dem röhrenförmigen Behälter verbleiben. Die Monomerflüssigkeit 4 wird durch den Filter 18, die Verbindung 14 und den Porenfilter 16 in das Zementpulver 5 gepresst und beginnt dort mit dem Zementpulver 5 zu reagieren, so dass sich aus dem Gemisch 54 der Knochenzementteig 54 bildet. Dabei kann die Monomerflüssigkeit 4 nicht direkt von dem Porenfilter 16 aus zur Innenwand der Kartusche 1 fließen, da diese vollständig oder im Fall eines geschlitzten Hohlzylinders 9 weitgehend von dem Hohlzylinder 9 abgedeckt ist. Dadurch wird die Monomerflüssigkeit 4 gezwungen sich einen Weg durch das Zementpulver 5 zu bahnen. Monomerflüssigkeitsblasen oder Monomerflüssigkeitsansammlungen können so verhindert werden und es wird ein homogenerer Knochenzementteig 54 gemischt als ohne Verwendung des Hohlzylinders 9. Die Menge der Monomerflüssigkeit 4 ist so gewählt, dass das Zementpulver 5 bis in die vorderste Spitze der Kartusche 1, das heißt, bis an den Verschluss 36 heran mit der Monomerflüssigkeit 4 benetzt wird. Der Verschluss 36 wird, sobald das Gemisch also der Knochenzementteig 54 entstanden ist, von dem auf den Knochenzementteig 54 aufgrund des Drucks auf den Austragskolben 7 wirkenden Druck nach vorne getrieben und in die Verschlussaufnahme 38 hineingedrückt, bis der Verschluss 36 auf den Anschlag 40 trifft, wo die Bewegung des Verschlusses 36 endet. Diese Situation ist in Figur 3, dritte Abbildung von oben und in den Detailansichten nach Figur 8 und Figur 9 oben sowie in den beiden Abbildungen unten rechts der Figur 6 gezeigt. Der Knochenzementteig 54 umfließt den Verschluss 36, indem er zwischen den Leisten 39 und zwischen den Leisten 40 hindurchfließt. Schließlich tritt der Knochenzementteig 54 an der Vorderseite der Vorrichtung aus.

In diesem Zustand (oder alternativ bereits zu Beginn) wird ein Applikatorrohr 66 auf das Außengewinde des Stutzens 42 als verlängerte Austragsöffnung oder in Form einer Austragsrohrverlängerung 70 aufgeschraubt (siehe auch Figur 5). Durch weiteres Vortreiben der Stange 44 wird der Förderkolben 6, die Scherben 52 und der davor angeordnete Austragskolben 7 angetrieben. Über das Applikatorrohr 66 wird dann der Knochenzementteig 54 aus der Kartusche 1 ausgetragen. Dazu wird der Austragskolben 7 mit der Stange 44 in Richtung des Leitungselements 37 vorgetrieben (siehe hierzu auch die vierte Abbildung von oben der Figur 3 sowie die Detailansicht nach Figur 8). Der Knochenzementteig 54 aus dem Inneren der Kartusche 1 wird durch den Stutzen 34, das Leitungselement 37 und das Applikatorrohr 66 ausgetrieben und kann dort appliziert werden oder theoretisch zur weiteren Verarbeitung verwendet werden.

Schließlich trifft der Hohlzylinder 9 auf den Kartuschenkopf beziehungsweise die Vorderseite des Innenraums der Kartusche 1. Da am Ende des Auspressvorgangs der Austragskolben 7 blockiert wird, kann es dazu kommen, dass die Scherben und Splitter 52 der Glasampulle 3 durch den auf die Scherben und Splitter 52 wirkenden wachsenden Druck noch weiter komprimiert werden und dabei noch weitere Reste der Monomerflüssigkeit 4 aus dem Zwischenraum zwischen dem Austragskolben 7 und dem Förderkolben 6 in den vorderen Teil der Kartusche 1 gedrückt werden. Dadurch kann es zu einer Veränderung der Zusammensetzung des Knochenzementteigs 54 kommen, da der Anteil an flüssiger Monomerflüssigkeit 4 in dem Knochenzementteig 54 erhöht wird. Wenn der Knochenzementteig 54 schon sehr weitgehend reagiert hat, kann es auch sein, dass sich die Monomerflüssigkeit 4 einen Weg an dem Knochenzementteig 54 vorbei bahnt. Der Hohlzylinder 9 weist eine Höhe von 3 mm, bevorzugt von 5 mm, oder größer auf, so dass durch den dadurch erzeugten Abstand gewährleistet ist, dass die Vorderseite des Austragskolbens 7 von der Vorderseite des Innenraums der Kartusche 1 beabstandet ist, wenn der Austragskolben 7 so weit nach vorne gedrückt es, wie es mit einer manuell angetriebenen Auspressvorrichtung 43 möglich ist. Dadurch entsteht im Innenraum der Kartusche 1 und zwar in dem von dem Hohlzylinder 9 begrenzten Bereich ein Totvolumen, das nicht aus der Kartusche 1 durch die Austragsöffnung und das Leitungselement 37 ausgetrieben werden kann.

In diesem Totvolumen befindet sich nun der Teil des Knochenzementteigs 54, der gegebenenfalls einen zu großen Anteil an Monomerflüssigkeit 4 enthält. Auch wenn nachfolgend immer weiter gepresst wird, kann kein weiterer Knochenzementteig 54 aus dem Totvolumen aus der Vorrichtung ausgepresst werden. Durch diesen Aufbau wird sichergestellt, dass kein Knochenzementteig 54 mit einer sich ändernden Konsistenz aufgrund sich ändernder Zusammensetzung mit der Vorrichtung appliziert werden kann.

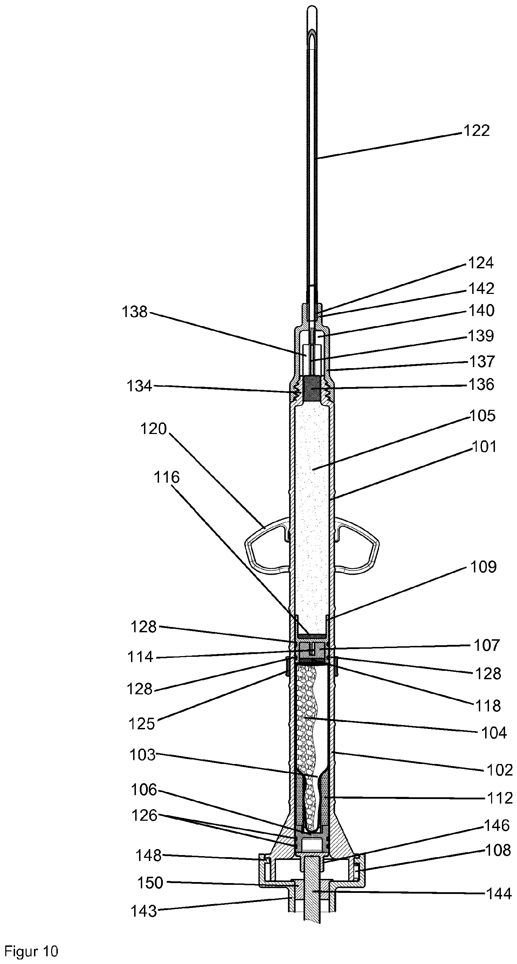

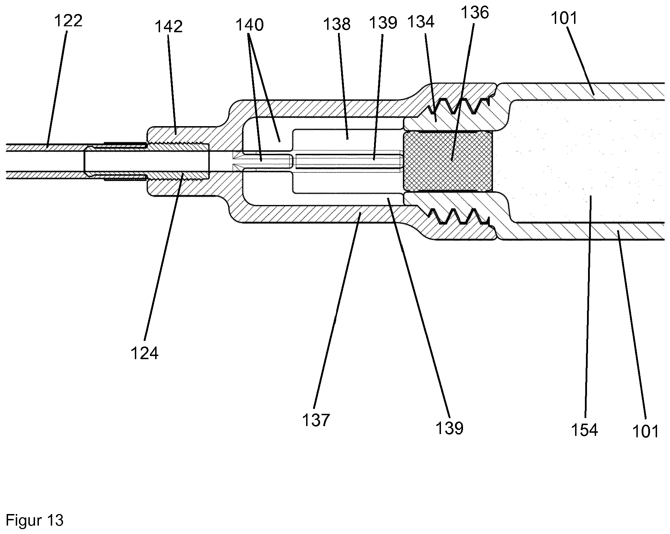

In den Figuren 10 bis 15 sind Abbildungen einer zweiten alternativen erfindungsgemäßen Vorrichtung gezeigt. Die Figuren 10 bis 12 zeigen verschiedene schematische Gesamtansichten der beispielhaften zweiten erfindungsgemäßen Vorrichtung. Die Figuren 13 bis 15 zeigen schematische Querschnittansichten als Detailansichten in Form von Ausschnittvergrößerungen durch verschiedene Bereiche der zweiten erfindungsgemäßen Vorrichtung. Die zweite erfindungsgemäße Vorrichtung ist ein sogenannter Spine-Applikator für die Spondylodese. Er wird zur Wirbelverblockung beziehungsweise Wirbelversteifung zweier Wirbelkörper eingesetzt, indem Knochenzementteig 154 unter Röntgenkontrolle mit Hilfe eines Trokars 120 im Bereich der Wirbel appliziert wird. Durch den Trokar 120 muss der Arzt nicht in der Röntgenstrahlung arbeiten.

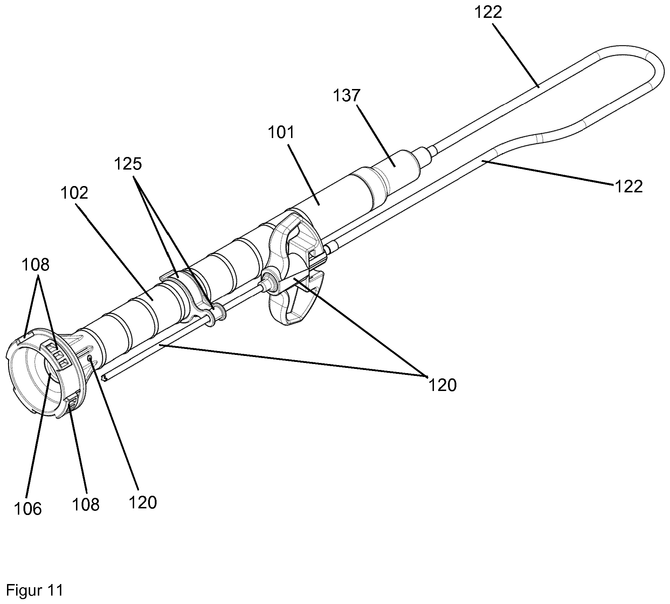

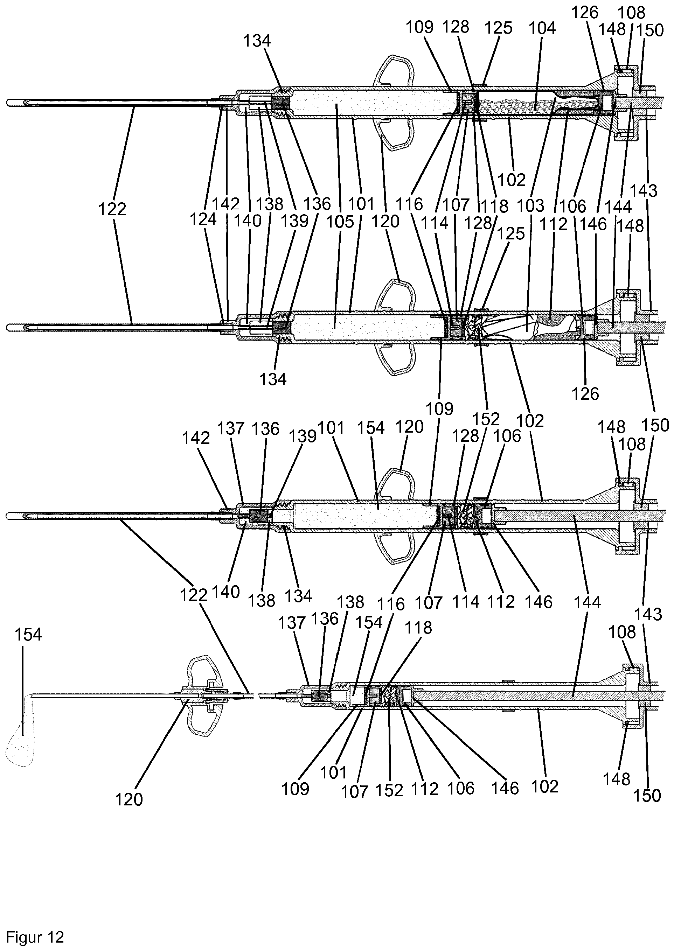

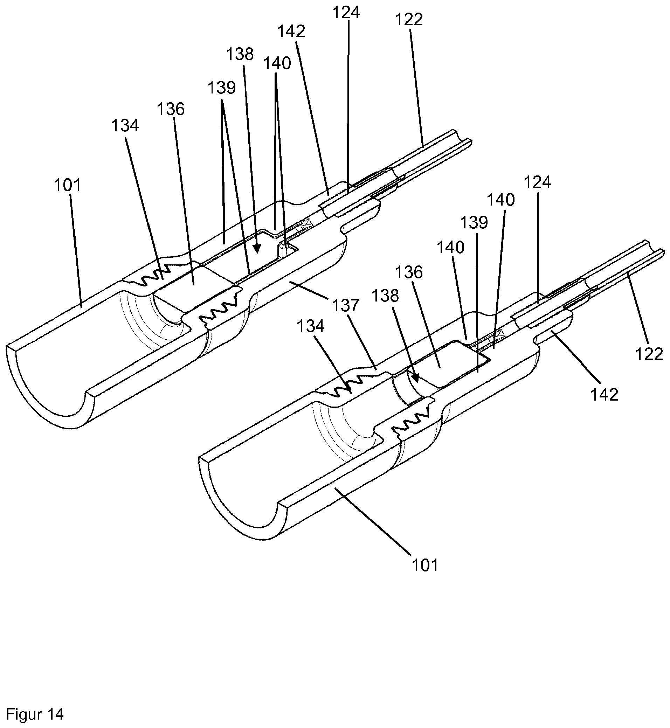

Die zweite erfindungsgemäße Vorrichtung ist weitgehend identisch zur ersten erfindungsgemäßen Vorrichtung nach den Figuren 1 bis 9 aufgebaut und besteht im Wesentlichen aus einem röhrenförmigen Behälter aus Kunststoff, der als vorderen Teil (in Figur 10 oben, in Figur 11 und 14 oben rechts in den Figuren 12, 13 und 15 links) eine Kartusche 101 mit einem zylindrischen Innenraum bildet und der als hinteren Teil eine Monomeraufnahme 102 für eine Glasampulle 103 als Monomerflüssigkeitsbehälter bildet. Anstelle der Glasampulle 103 kann auch ohne weiteres eine aufbrechbare Kunststoffampulle verwendet werden oder es kann durch geringe Umbaumaßnahmen auch ein aufreißbarer Folienbeutel aus einem Metall-beschichteten Kunststoff anstelle der Glasampulle 103 verwendet werden.

Die Rückseite der Vorrichtung ist in Figur 10 unten, in den Abbildungen der Figur 12 rechts und in Figur 11 unten links gezeigt. Die Röhrenform des Behälters ist besonders gut in den Querschnittansichten der Figuren 10 und 12 sowie der perspektivischen Ansicht nach Figur 11 zu erkennen. Sowohl der Innenraum der Kartusche 101 als auch der Innenraum der Monomeraufnahme 102 sind zylindrisch mit kreisförmiger Grundfläche. Dabei sind die Durchmesser des Innenraums der Kartusche 101 und der Durchmesser des Innenraums der Monomeraufnahme 102 gleich groß und fluchten. Der Behälter mit der Monomeraufnahme 102 und der Kartusche 101 wird vorzugsweise mit Hilfe einer Spritzgusstechnik aus Kunststoff hergestellt. Die Monomeraufnahme 102 weist also einen zylindrischen Innenraum auf, in den die Glasampulle 103 eingesteckt ist. In der Glasampulle 103 befindet sich die Monomerflüssigkeit 104. In Figur 10 ist die Vorrichtung gekippt gezeigt, so dass die Gravitation zur Seite wirkt und sich die Monomerflüssigkeit 104 auf einer Seite der Glasampulle 103 sammelt. In den Innenraum der Kartusche 101 ist ein Zementpulver 105 eingefüllt oder vorzugsweise eingepresst. Die Monomerflüssigkeit 104 und das Zementpulver 105 bilden die Ausgangskomponenten für einen PMMA-Knochenzement, der mit der Vorrichtung herstellbar ist. Aufgrund der Glasampulle 103 kann die Monomerflüssigkeit 104 sehr lange in der Monomeraufnahme 102 und dadurch in der Vorrichtung gelagert werden. Das Zementpulver 105 kann ebenfalls über längere Zeiträume in der Vorrichtung gelagert werden. Die Vorrichtung ist damit zum Lagern der Monomerflüssigkeit 104 und des Zementpulvers 105 als Ausgangskomponenten eines Knochenzementteigs 154 des PMMA-Knochenzements geeignet. Die Vorrichtung ist aber auch zum Mischen des Knochenzementteigs 154 aus den Ausgangskomponenten 104, 105 und zum Austragen des gemischten Knochenzementteigs 154 geeignet und vorgesehen.

In der Monomeraufnahme 102 ist ein im zylindrischen Innenraum der Monomeraufnahme 102 in Längsrichtung beweglicher Förderkolben 106 aus Kunststoff angeordnet. Der Förderkolben 106 ist im Bereich der Rückseite der Monomeraufnahme 102 angeordnet. Die Glasampulle 103 kann mit dem Förderkolben 106 in der Monomeraufnahme 102 zusammengedrückt und dabei zersplittert werden, indem der Förderkolben 106 in Richtung der Vorderseite, also in Richtung der Kartusche 101 gedrückt wird. Der Förderkolben 106 weist an der Vorderseite Abstreifer auf, mit denen Splitter der Glasampulle 103 von der Innenwand der Monomeraufnahme 102 abgestreift werden. Die Abstreifer liegen hierzu seitlich an der Innenwand des Innenraums der Monomeraufnahme 102 an. Ferner ist an der Vorderseite des Förderkolbens 106 eine Kante 180 angeordnet, die ein Aufbrechen der Glasampulle 103 beim Vortreiben des Förderkolbens 106 in der Monomeraufnahme 102 erleichtert.

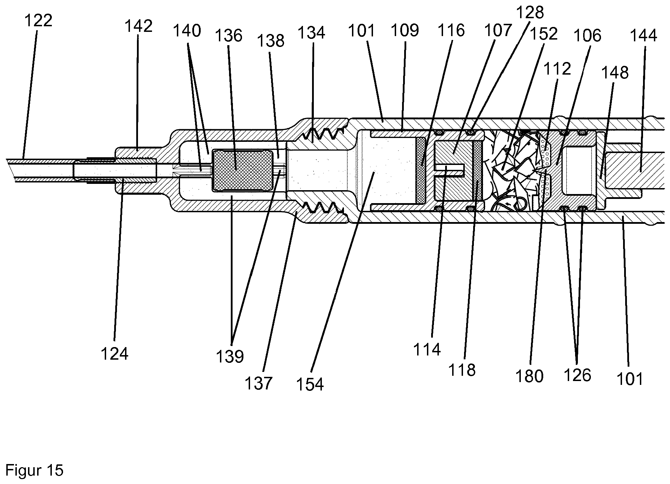

In dem Innenraum der Kartusche 101 ist in dessen Rückseite (in Figur 10 Richtung unten, in den Figuren 12 und 15 Richtung rechts) ein Austragskolben 107 aus Kunststoff angeordnet. An der Rückseite der Monomeraufnahme 102 ist ein Befestigungsmittel 108 vorgesehen, mit dem die Monomeraufnahme 102 beziehungsweise der Behälter an einer Auspressvorrichtung 143 (in Figur 11 nicht zu sehen, siehe aber Figuren 10 und 12) verbunden werden kann. Das Befestigungsmittel 108 ist vorzugsweise zur Bildung eines Bajonettverschlusses 108 geeignet und vorgesehen. Dadurch kann der Förderkolben 106, der von der Rückseite der Monomeraufnahme 102 aus frei zugänglich ist, mit der Auspressvorrichtung 143 in Richtung der Vorderseite der Kartusche 101 vorgetrieben werden.

Der Austragskolben 107 weist an seiner Vorderseite einen Hohlzylinder 109 zum Verlängern der Strecke auf, die die Monomerflüssigkeit 104 durch das Zementpulver 105 fließen muss, bis sie zur Innenwand der Kartusche 101 gelangt. Zudem dient der Hohlzylinder 109 zur Beabstandung des Austragskolbens 107 von einer Austragsöffnung an der Vorderseite des Innenraums der Kartusche 101 sowie zum Schaffen eins Totvolumens zwischen dem Austragskolben 107 und der Vorderseite des Innenraums der Kartusche 101, wenn der Austragskolben 107 beziehungsweise der Hohlzylinder 109 maximal an die Vorderseite des Innenraums der Kartusche 101 gedrückt ist. Der Hohlzylinder 109 ist vorliegend rotationssymmetrisch und ist nach Art eines Rohrstücks geformt. Der Hohlzylinder 109 kann aber auch parallel zur Zylinderachse des Hohlzylinders 109 verlaufende Längsschnitte aufweisen. An der Vorderseite ist der Hohlzylinder 109 eben.

In dem Innenraum der Monomeraufnahme 102 ist eine Lagerung 112 aus Schaumstoff vorgesehen, die als Transportsicherung und als Stoßsicherung für die Glasampulle 103 dient. Damit soll verhindert werden, dass die Glasampulle 103 bei Erschütterungen oder Stößen ungewollt aufbricht. Der Schaumstoff und damit die Lagerung 112 sind durchlässig für Gase.

Die Kartusche 101 und die Monomeraufnahme 102 sind einteilig als gemeinsames Kunststoffteil ausgeführt. Die Monomeraufnahme 102 und die Kartusche 101 sind für die Monomerflüssigkeit 104 flüssigkeitsdurchlässig miteinander über eine Verbindung 114 im Austragskolben 107 verbunden. Die Verbindung 114 durch den Austragskolben 107 mündet durch einen für das Zementpulver 105 undurchlässigen aber für die Monomerflüssigkeit 104 durchlässigen Porenfilter 116 in den Innenraum der Kartusche 101.

In der Einmündung zur Verbindung 114 ist in dem Austragskolben 107 ein Filter 118 angeordnet, mit dem die Splitter 152 der Glasampulle 103 zurückgehalten werden können. Statt dem Filter 118 oder zusätzlich zum Filter 118 kann auch ein Sieb vorgesehen sein.

Mehrere Belüftungsöffnungen 120 sind in der Wandung der Monomeraufnahme 102 vorgesehen, durch die der Innenraum der Monomeraufnahme 102 mit Hilfe eines sterilisierenden Gases wie Ethylenoxid sterilisiert werden kann. Die Lagerung 112 ist ebenfalls gasdurchlässig und verschließt daher nicht die Belüftungsöffnungen 120. Die Belüftungsöffnungen 120 sind unmittelbar benachbart zum Förderkolben 106 angeordnet, so dass der Förderkolben 106 sich unmittelbar vor die Belüftungsöffnungen 120 schiebt und somit die Belüftungsöffnungen 120 unmittelbar verschließt, wenn der Förderkolben 106 in Richtung der Kartusche 101 vorgetrieben wird. Dadurch wird verhindert, dass Monomerflüssigkeit 104 durch die Belüftungsöffnungen 120 austreten kann, wenn die Glasampulle 103 in der Monomeraufnahme 102 geöffnet wurde.

Der zylindrische Förderkolben 106 hat einen zur Zylindergeometrie des Innenraums der Monomeraufnahme 102 passenden Außenumfang und ist über zwei umlaufende Dichtungen 126 gegen die Innenwand der Monomeraufnahme 102 flüssigkeitsdicht abgedichtet. Ebenso ist der Austragskolben 107 über zwei umlaufende Dichtungen 128 gegen die Innenwand der Kartusche 101 flüssigkeitsdicht abgedichtet. Diese Dichtungen 126, 128 dienen dazu, einen Austritt von Monomerflüssigkeit 104 oder von Knochenzement zu verhindern, um eine Kontamination der Umgebung (des OP-Saals und des Anwenders) zu verhindern. Die Dichtungen 126, 128 können hierzu aus Gummi bestehen.

Der Innenraum der Kartusche 101 mündet an der Vorderseite in einen Stutzen 134, der die Austragsöffnung der Kartusche 101 begrenzt. Der Stutzen 134 weist ein Außengewinde auf. Im Inneren des Stutzens 134 ist ein Verschluss 136 für die Kartusche 101 angeordnet, der in der Austragsöffnung steckt und diese verschließt. Der Verschluss 136 ist ein für das Zementpulver 105 undurchlässiger aber für Gase durchlässiger Porenfilter und hat eine zylindrische Form.

An dem Außengewinde des Stutzens 134 ist ein Leitungselement 137 mit einer Verschlussaufnahme 138 zur Aufnahme des Verschlusses 136 befestigt. Die Verschlussaufnahme 138 ist nach Art einer Hülse geformt und weist vier in Längsrichtung ausgerichtete, sich in die Verschlussaufnahme 138 hinein erhebende Leisten 139 auf. Die Leisten 139 beabstanden den Verschluss 136 von der Innenwand der Verschlussaufnahme 138, wenn der Verschluss 136 in die Verschlussaufnahme 138 hineingedrückt ist. Vor der Verschlussaufnahme 138 verengt sich das Leitungselement 137. In diesem Bereich sind vier weitere Leisten 140 angeordnet, die einen Anschlag 140 für die Bewegung des Verschlusses 136 bilden und also die Bewegung des Verschlusses 136 in die Verschlussaufnahme 138 begrenzen. Zwischen den Leisten 139, 140 ist ein ausreichender freier Leitungsquerschnitt vorgesehen, so dass der Knochenzementteig 154 zwischen den Leisten 139, der Wandung der Verschlussaufnahme 138 und dem eingeschobenen Verschluss 136 sowie zwischen den Leisten 140 im vorderen Teil des Leitungselements 137 hindurch strömen kann. An seiner Vorderseite endet das Leitungselement 137 in einem Stutzen 142 mit einem Innengewinde.

An das Leitungselement 137 ist ein Trokar 120 und ein Schlauch 122 zum verbinden des Trokars 120 angeschlossen. Hierzu ist in das Innengewinde des Stutzens 142 Einsatz 124 mit einem passenden Außengewinde eingeschraubt, wobei der Einsatz mit dem Schlauch 122 über eine Krimp-Verbindung verbunden ist. Auch der Trokar 120 ist mit dem Schlauch 122 über eine Krimp-Verbindung verbunden. An dem Behälter 101, 102 ist außen eine Halterung 125 befestigt, an die eine Applikationsspitze des Trokars 120 angeklemmt werden kann, damit der Trokar 120 nicht lose an der Vorrichtung herumbaumelt.

Durch den als Porenfilter ausgeführten Verschluss 136 hindurch können das Innere der Kartusche 101 und das Zementpulver 105 mit Hilfe von Ethylenoxid sterilisiert werden, da das Leitungselement 137 offen ist, der Trokar 120 mit dem Schlauch 122 beim Sterilisieren noch nicht angeschlossen ist und der Verschluss 136 und die Zwischenräume zwischen den Pulverpartikeln des Zementpulvers 105 luftdurchlässig sind. Gleichzeitig kann Luft aus der Monomeraufnahme 102 durch das Zementpulver 105, den Verschluss 136, das Leitungselement 137, den Schlauch 122 und den Trokar 120 herausgepresst werden, wenn der Förderkolben 106 in Richtung des Austragskolbens 107 gepresst wird.

Das Zementpulver 105 ist in der Kartusche 101 eingeschlossen, da alle Öffnungen und Verbindungen 114 mit Hilfe der Porenfilter 116, 136 für das Zementpulver 105 undurchlässig verschlossen sind. Der Inhalt der Kartusche 101 kann dabei durch Evakuieren und Spülen mit Ethylenoxid sterilisiert werden. Dadurch ist die Vorrichtung auch zum langfristigen Lagern des Zementpulvers 105 geeignet.

Figur 14 zeigt zwei schematische perspektivische Querschnittansichten des vorderen Teils der Vorrichtung im geschlossenen Zustand (Abbildung auf der linken Seite) und im geöffneten Zustand (Abbildung auf der rechten Seite). Im geöffneten Zustand ist der Verschluss 136 analog dem ersten Ausführungsbeispiel mit den Leisten 139 von der Innenwand der Verschlussaufnahme 138 beabstandet. Dadurch bildet sich dazwischen ein freier Leitungsquerschnitt durch den der Knochenzementteig 154 durch das Leitungselement 137, durch den Schlauch 122 und durch den Trokar 120 aus der Vorrichtung herausgepresst werden kann.

Figur 12 zeigt vier schematische Querschnittansichten der zweiten erfindungsgemäßen Vorrichtung übereinander zur Verdeutlichung des Ablaufs eines erfindungsgemäßen Verfahrens. Dazu zeigt Figur 13 eine Ausschnittvergrößerung der ersten Abbildung von oben der Figur 12 und Figur 15 eine Ausschnittvergrößerung der vierten Abbildung von oben der Figur 12. Die Abbildung in Figur 14 rechts zeigt eine Ausschnittvergrößerung der dritten Abbildung von oben der Figur 12 und die Abbildung in Figur 14 links zeigt eine Ausschnittvergrößerung der zweiten Abbildung von oben der Figur 12.

Zu Beginn des Verfahrens liegt die Vorrichtung im Ausgangszustand vor, so wie sie auch in Figur 10 gezeigt ist. In diesem Zustand wird die Vorrichtung in die Auspressvorrichtung 143 eingesetzt, beispielsweise eine herkömmliche mit der Hand manuell antreibbare Kartuschenpistole. Diese Situation ist in der obersten Abbildung von Figur 12 gezeigt. Die Auspressvorrichtung 143 umfasst eine linear vortreibbare Stange 144. Von der Auspressvorrichtung 143 ist nur der vordere Teil dargestellt. Die Auspressvorrichtung 143 umfasst auch einen Griff und einen Kipphebel (in den Abbildungen nicht zu sehen) zum manuellen Antreiben der Stange 144 der Auspressvorrichtung 143, wie bei herkömmlichen manuell angetriebenen Auspressvorrichtungen auch. Die Vorrichtung wird mit dem Befestigungsmittel 108 an der Auspressvorrichtung 143 befestigt (siehe oberste Abbildung in Figur 12). An der Spitze der Stange 144 ist ein flacher Teller 146 zum Antreiben des Förderkolbens 106 vorgesehen. Die Stange 144 drückt mit dem Teller 146 auf den Förderkolben 106, wenn die Stange 144 von der Auspressvorrichtung 143 in die Monomeraufnahme 102 hineingedrückt wird. Die Auspressvorrichtung 143 ist hierzu über ein Gegenbefestigungsmittel 148 an die Rückseite der Monomeraufnahme 102 angeschlossen, so dass der Teller 146 beim Vortreiben der Stange 144 auf den Förderkolben 106 drückt und diesen in Richtung der Kartusche 101 vortreibt. Hierzu ist die Stange 144 gegen ein Lager 150 und darüber gegen das Gegenbefestigungsmittel 148 und damit gegen die Monomeraufnahme 102 linear beweglich gelagert.

Die Auspressvorrichtung 143 wird bedient und dabei die Stange 144 und mit der Stange 144 der Förderkolben 106 in Richtung der Kartusche 101 vorgetrieben. Zu Beginn der Bewegung des Förderkolbens 106 verschließt dieser die Belüftungsöffnungen 120. Die Lagerung 112 wird komprimiert und der Förderkolben 106 trifft auf den Kopf der Glasampulle 103. Da die Glasampulle 103 an der Vorderseite an dem Austragskolben 107 anliegt und sich der Innenraum der Monomeraufnahme 102 weiter verkleinert, wird die Glasampulle 103 zerbrochen. Die Monomerflüssigkeit 104 tritt aus der Glasampulle 103 in den Innenraum der Monomeraufnahme 102 aus. Der Austragskolben 107 kann nicht oder nicht weit von der Glasampulle 103 in Richtung des Verschlusses 136 geschoben werden, wenn das Zementpulver 105 trocken ist, also nicht von der Monomerflüssigkeit 104 benetzt ist, da das trockene Zementpulver 105 nicht fließfähig ist und eine Bewegung des Austragskolbens 107 blockiert. Diese Situation ist in Figur 12, zweite Abbildung von oben, sowie in der vergrößerten Ausschnittansicht in Figur 13 gezeigt. Überstehende Luft aus der Monomeraufnahme 102 wird durch den Filter 118, die Verbindung 114, den Porenfilter 116, durch die Zwischenräume zwischen den Partikeln des Zementpulvers 105, durch den Verschluss 136, durch das Leitungselement 137, durch den Schlauch 122 und den Trokar 120 aus der Vorrichtung herausgedrückt.

Von der Glasampulle 103 bleiben schließlich nur kleine Splitter 152 zurück, die von dem Filter 118 zurückgehalten werden und in dem röhrenförmigen Behälter verbleiben. Die Monomerflüssigkeit 104 wird durch den Filter 118, die Verbindung 114 und den Porenfilter 116 in das Zementpulver 105 gepresst und beginnt dort mit dem Zementpulver 105 zu reagieren, so dass sich aus dem Gemisch der Knochenzementteig 154 bildet. Dabei kann die Monomerflüssigkeit 104 nicht direkt von dem Porenfilter 116 aus zur Innenwand der Kartusche 101 fließen, da diese vollständig oder im Fall eines geschlitzten Hohlzylinders 109 weitgehend von dem Hohlzylinder 109 abgedeckt ist. Dadurch wird die Monomerflüssigkeit 104 gezwungen sich einen Weg durch das Zementpulver 105 zu bahnen. Monomerflüssigkeitsblasen oder Monomerflüssigkeitsansammlungen können so verhindert werden und es wird ein homogenerer Knochenzementteig 154 gemischt als ohne Verwendung des Hohlzylinders 109.

Die Menge der Monomerflüssigkeit 104 ist so gewählt, dass das Zementpulver 105 bis in die vorderste Spitze der Kartusche 101, das heißt, bis an den Verschluss 136 heran mit der Monomerflüssigkeit 104 benetzt wird. Der Verschluss 136 wird, sobald das Gemisch also der Knochenzementteig 154 entstanden ist, von dem auf den Knochenzementteig 154 aufgrund des Drucks auf den Austragskolben 107 wirkenden Druck nach vorne getrieben und in die Verschlussaufnahme 138 hineingedrückt, bis der Verschluss 136 auf den Anschlag 140 trifft, wo die Bewegung des Verschlusses 136 endet. Diese Situation ist in Figur 12, dritte Abbildung von oben und in der Detailansicht nach Figur 14 rechts gezeigt. Der Knochenzementteig 154 umfließt den Verschluss 136, indem er zwischen den Leisten 139 und zwischen den Leisten 140 hindurchfließt. Anschließend wird der Knochenzementteig 154 durch den Schlauch 122 und in den Trokar 120 gepresst. Aus dem Trokar 120 kann der Knochenzementteig 154 an der Vorderseite der Vorrichtung ausfließen.

Durch weiteres Vortreiben der Stange 144 wird der Förderkolben 106, die Scherben 152 und der davor angeordnete Austragskolben 107 angetrieben. Über den Schlauch 122 und den Trokar 120 wird dann der Knochenzementteig 154 aus der Kartusche 101 ausgetragen. Dazu wird der Austragskolben 107 mit der Stange 144 in Richtung des Leitungselements 137 vorgetrieben (siehe hierzu auch die vierte Abbildung von oben der Figur 12 sowie die Detailansicht nach Figur 15).

Schließlich trifft der Hohlzylinder 109 auf den Kartuschenkopf beziehungsweise die Vorderseite des Innenraums der Kartusche 101. Da am Ende des Auspressvorgangs der Austragskolben 107 blockiert wird, kann es dazu kommen, dass die Scherben und Splitter 152 der Glasampulle 103 durch den auf die Scherben und Splitter 152 wirkenden wachsenden Druck noch weiter komprimiert werden und dabei noch weitere Reste der Monomerflüssigkeit 104 aus dem Zwischenraum zwischen dem Austragskolben 107 und dem Förderkolben 106 in den vorderen Teil der Kartusche 101 gedrückt werden. Dadurch kann es zu einer Veränderung der Zusammensetzung des Knochenzementteigs 154 kommen, da der Anteil an flüssiger Monomerflüssigkeit 104 in dem Knochenzementteig 154 erhöht wird. Wenn der Knochenzementteig 154 schon sehr weitgehend reagiert hat, kann es auch sein, dass sich die Monomerflüssigkeit 104 einen Weg an dem Knochenzementteig 154 vorbei bahnt. Der Hohlzylinder 109 weist eine Höhe von 3 mm, bevorzugt von 5 mm, oder größer auf, so dass durch den dadurch erzeugten Abstand gewährleistet ist, dass die Vorderseite des Austragskolbens 107 von der Vorderseite des Innenraums der Kartusche 1 beabstandet ist, wenn der Austragskolben 107 so weit nach vorne gedrückt es, wie es mit einer manuell angetriebenen Auspressvorrichtung 143 möglich ist. Dadurch entsteht im Innenraum der Kartusche 101 und zwar in dem von dem Hohlzylinder 109 begrenzten Bereich ein Totvolumen, das nicht aus der Kartusche 101 durch die Austragsöffnung, den Schlauch 122 und den Trokar 120 ausgetrieben werden kann.

In diesem Totvolumen befindet sich nun der Teil des Knochenzementteigs 154, der gegebenenfalls einen zu großen Anteil an Monomerflüssigkeit 104 enthält. Auch wenn nachfolgend immer weiter gepresst wird, kann kein weiterer Knochenzementteig 154 aus dem Totvolumen aus der Vorrichtung ausgepresst werden. Durch diesen Aufbau wird sichergestellt, dass kein Knochenzementteig 154 mit einer sich ändernden Konsistenz aufgrund sich ändernder Zusammensetzung mit der Vorrichtung appliziert werden kann.

### Bezugszeichenliste

- 1, 101: Kartusche
- 2, 102: Monomeraufnahme
- 3, 103: Ampulle
- 4, 104: Monomerflüssigkeit
- 5, 105: Zementpulver
- 6, 106: Förderkolben
- 7, 107: Austragskolben
- 8, 108: Befestigungsmittel / Bajonettverschluss
- 9, 109: Hohlzylinder
- 12, 112: Lagerung
- 14, 114: Verbindung
- 16, 116: Porenfilter
- 18, 118: Filter
- 20, 120: Belüftungsöffnung
- 26, 126: Dichtung
- 28, 128: Dichtung
- 34, 134: Anschluss
- 36, 136: Verschluss / Porenfilter
- 37, 137: Leitungselement
- 38, 138: Verschlussaufnahme
- 39, 139: Leiste / Abstandhalter
- 40, 140: Anschlag / Leiste
- 42: Stutzen mit Außengewinde
- 43, 143: Auspressvorrichtung / Kartuschenpistole
- 44, 144: Stange
- 46, 146: Teller
- 48, 148: Gegenbefestigungsmittel / Bajonettverschluss
- 50, 150: Lagerung
- 52, 152: Splitter
- 54, 154: Knochenzementteig
- 66: Applikatorrohr
- 70: Austragsrohrverlängerung
- 72: Verschluss
- 74: Griff
- 76: Freier Leitungsquerschnitt
- 120: Trokar
- 122: Schlauch
- 124: Einsatz
- 125: Halterung
- 142: Stutzen mit Innengewinde
- 180: Kante
- A-A: Schnittebene

## Patentansprüche

1. Vorrichtung zum Herstellen eines Knochenzementteigs (54, 154) aus einer Monomerflüssigkeit (4, 104) und einem Zementpulver (5, 105) als Ausgangskomponenten des Knochenzementteigs (54, 154) und zum Austragen des gemischten Knochenzementteigs (54, 154), die Vorrichtung aufweisend
eine Kartusche (1, 101) mit einem zylindrischen Innenraum, in dem die Ausgangskomponenten gemischt werden, wobei der Innenraum der Kartusche (1, 101) an der Vorderseite bis auf eine Austragsöffnung zum Austreiben des Knochenzementteigs (54, 154) aus dem Innenraum geschlossen ist,
einen Austragskolben (7, 107), der im Innenraum der Kartusche (1, 101) angeordnet ist und der in Richtung der Austragsöffnung drückbar gelagert ist,
das Zementpulver (5, 105), das im Innenraum der Kartusche (1, 101) zwischen der Austragsöffnung und dem Austragskolben (7, 107) angeordnet ist,
einen Verschluss (36, 136), der die Austragsöffnung verschließt und der gegen die Austragsöffnung beweglich gelagert ist, und
ein Leitungselement (37, 137), das an der Vorderseite der Austragsöffnung angeordnet ist, wobei das Leitungselement (37, 137) eine Verschlussaufnahme (38, 138) zum Aufnehmen zumindest eines Teils des Verschlusses (36, 136) umfasst,
wobei der Verschluss (36, 136) durch einen Druck auf den Knochenzementteig (54, 154) derart in die Verschlussaufnahme (38, 138) hinein drückbar ist, dass die Austragsöffnung geöffnet ist, wobei das Leitungselement (37, 137) mit dem in die Verschlussaufnahme (38, 138) gedrückten Verschluss (36, 136) einen freien Leitungsquerschnitt (76) bereitstellt, durch den der Knochenzementteig (54, 154) aus der Austragsöffnung hindurch und aus der Vorrichtung heraus drückbar ist, wobei
in dem Austragskolben (7, 107) zumindest eine Verbindung (14, 114) von der Rückseite des Austragskolbens (7, 107) zur Vorderseite des Austragskolbens (7, 107) zum Einleiten der Monomerflüssigkeit (4, 104) in den Innenraum der Kartusche (1, 101) vorgesehen ist, wobei die zumindest eine Verbindung (14, 114) für die Monomerflüssigkeit (4, 104) und Gase durchlässig ist und für das Zementpulver (5, 105) undurchlässig ist.

2. Vorrichtung nach Anspruch 1, **dadurch gekennzeichnet, dass**
der Knochenzementteig (54, 154) den Verschluss (36, 136) in der Verschlussaufnahme (38, 138) umfließt, wenn der Knochenzementteig (54, 154) durch das Leitungselement (37, 137) fließt, vorzugsweise der Knochenzementteig (54, 154) entlang wenigstens einer Seitenfläche oder Mantelfläche des Verschlusses (36, 136) an dem Verschluss (36, 136) vorbeifließt.

3. Vorrichtung nach einem der vorangehenden Ansprüche, **dadurch gekennzeichnet, dass**
der Verschluss (36, 136) fest in der Verschlussaufnahme (38, 138) steckt, wenn er aus der Austragsöffnung in die Verschlussaufnahme (38, 138) hineingedrückt ist und/oder der freie Leitungsquerschnitt (76) auf einer Seite zumindest bereichsweise durch den Verschluss (36, 136) begrenzt ist, vorzugsweise durch eine Seitenfläche oder eine Mantelfläche des Verschlusses (36, 136) begrenzt ist.

4. Vorrichtung nach einem der vorangehenden Ansprüche, **dadurch gekennzeichnet, dass**
der Verschluss (36, 136) zumindest bereichsweise zylindrisch ist, insbesondere vollständig zylindrisch ist, und die Verschlussaufnahme (38, 138) eine hohlzylinderförmige Hülse bildet, wobei in der Mantelfläche der hohlzylinderförmigen Hülse zumindest ein Kanal vorgesehen, wobei der zumindest eine Kanal den freien Leitungsquerschnitt (76) bereitstellt, wobei vorzugsweise
der Innendurchmesser der hohlzylinderförmigen Hülse größer ist als der Außendurchmesser des Verschlusses (36, 136), vorzugsweise wenigstens 1 mm größer ist als der Außendurchmesser des Verschlusses (36, 136), besonders bevorzugt zwischen 1 mm und 10 mm größer ist als der Außendurchmesser des Verschlusses (36, 136) und/oder
die axiale Länge des Innenraums der hohlzylinderförmigen Hülse größer ist als die axiale Länge des Verschlusses (36, 136), vorzugsweise wenigstens 1 mm größer ist als die axiale Länge des Verschlusses (36, 136), besonders bevorzugt zwischen 1 mm und 20 mm größer ist als die axiale Länge des Verschlusses (36, 136).

5. Vorrichtung nach einem der vorangehenden Ansprüche, **dadurch gekennzeichnet, dass**
in der Verschlussaufnahme (38, 138) Abstandhalter (39, 40, 139, 140) zur Beabstandung des Verschlusses (36, 136) von der Innenwand der Verschlussaufnahme (38, 138) vorgesehen sind, wobei vorzugsweise die Abstandhalten Leisten (39, 40, 139, 140) sind, die besonders bevorzugt in der Bewegungsrichtung des Verschlusses (36, 136) ausgerichtet sind und/oder die in Flussrichtung des Knochenzementteigs (54, 154) ausgerichtet sind.

6. Vorrichtung nach einem der vorangehenden Ansprüche, **dadurch gekennzeichnet, dass**
die Länge des Verschlusses (36, 136) in seiner Bewegungsrichtung größer ist, als die Breite in den Richtungen senkrecht dazu und/oder
der freie Leitungsquerschnitt (76) zumindest halb groß ist wie der Querschnitt der Austragsöffnung, vorzugsweise zumindest so groß ist wie der Querschnitt der Austragsöffnung.

7. Vorrichtung nach einem der vorangehenden Ansprüche, **dadurch gekennzeichnet, dass**
in der Verschlussaufnahme (38, 138) an der von der Austragsöffnung abgewandten vorderen Stirnwand ein Anschlag (40, 140) zur Begrenzung der Bewegung des Verschlusses (36, 136) angeordnet ist, wobei der Anschlag (40, 140) den Verschluss (36, 136) im vollständig eingedrücktem Zustand von der Stirnwand an der Vorderseite der Verschlussaufnahme (38, 138) beabstandet, so dass zwischen der Vorderseite des Verschlusses (36, 136) und der vorderen Stirnwand der freie Leitungsquerschnitt (76) verbleibt.

8. Vorrichtung nach einem der vorangehenden Ansprüche, **dadurch gekennzeichnet, dass**
die Vorrichtung eine Monomeraufnahme (2, 102) aufweist, in der die Monomerflüssigkeit (4, 104), insbesondere ein Monomerflüssigkeitsbehälter (3, 103) enthaltend die Monomerflüssigkeit (4, 104), enthalten ist, wobei die Rückseite der Kartusche (1, 101) mit der Vorderseite der Monomeraufnahme (2, 102) verbunden ist, vorzugsweise derart verbunden ist, dass der Innenraum der Kartusche (1, 101) mit dem Innenraum der Monomeraufnahme (2, 102) fluchtet, wobei ein Innenraum der Monomeraufnahme (2, 102) und der Innenraum der Kartusche (1, 101) über eine für die Monomerflüssigkeit (4, 104) und für Gase durchlässige aber für das Zementpulver (5, 105) undurchlässige Verbindung (14, 114) miteinander verbunden sind und/oder
die Monomeraufnahme (2, 102) einen zylindrischen Innenraum aufweist, in dem die Monomerflüssigkeit (4, 104), insbesondere ein Monomerflüssigkeitsbehälter (3, 103) enthaltend die Monomerflüssigkeit (4, 104), angeordnet ist.

9. Vorrichtung nach Anspruch 8, **dadurch gekennzeichnet, dass**
in der Wandung der Monomeraufnahme (2, 102) zumindest eine Belüftungsöffnung (20, 120) angeordnet ist, die den Innenraum der Monomeraufnahme (2, 102) mit der Umgebung verbindet, und/oder
in der Monomeraufnahme (2, 102) ein in Längsrichtung der Monomeraufnahme (2, 102) beweglicher Förderkolben (6, 106) angeordnet ist, der von einer Rückseite der Monomeraufnahme (2, 102) aus in Richtung der Vorderseite vortreibbar ist, wobei die Monomerflüssigkeit (4, 104), insbesondere ein Monomerflüssigkeitsbehälter (3, 103) enthaltend die Monomerflüssigkeit (4, 104), zwischen dem Förderkolben (6, 106) und dem Austragskolben (7, 107) angeordnet ist.

10. Vorrichtung nach einem der vorangehenden Ansprüche, **dadurch gekennzeichnet, dass**
an der Rückseite der Vorrichtung, ein Befestigungsmittel (8, 108) zur Befestigung einer Auspressvorrichtung (43, 143) angeordnet ist, mit der der Austragskolben (7, 107) in Richtung der Austragsöffnung drückbar ist.

11. Vorrichtung nach einem der vorangehenden Ansprüche, **dadurch gekennzeichnet, dass**
an der der Austragsöffnung zugewandten Vorderseite des Austragskolbens (7, 107) ein Hohlzylinder (9, 109) angeordnet ist, wobei der Hohlzylinder (9, 109) an seiner der Austragsöffnung zugewandten Vorderseite offen ist und der sich vorzugsweise von der Vorderseite des Austragskolbens (7, 107) zumindest 3 mm in den Innenraum der Kartusche (1, 101) erstreckt, wobei
der Hohlzylinder (9, 109) eine weitere Bewegung des Austragskolbens (7, 107) in Richtung der Vorderseite der Kartusche (1, 101) blockiert, wenn die Vorderseite des Hohlzylinders (9, 109) an der Vorderseite des Innenraums der Kartusche (1, 101) anliegt, so dass der Austragskolben (7, 107) von der Vorderseite des Innenraums der Kartusche (1, 101) beabstandet ist und ein Totvolumen in dem Innenraum der Kartusche (1, 101) verbleibt.

12. Vorrichtung nach einem der vorangehenden Ansprüche, **dadurch gekennzeichnet, dass**
die zumindest eine Verbindung (14, 114) aus dem Austragskolben (7, 107) innerhalb des Hohlzylinders (9, 109) oder durch Leitungen in dem Hohlzylinder (9, 109) an der Vorderseite des Hohlzylinders (9, 109) in den Innenraum der Kartusche (1, 101) münden, und
der Verschluss (36, 136) an der dem Innenraum der Kartusche (1, 101) zugewandten Rückseite eine Vertiefung aufweist, in der der vorderste Teil des Zementpulvers (5, 105) enthalten ist.

13. Vorrichtung nach einem der vorangehenden Ansprüche, **dadurch gekennzeichnet, dass**
das Volumen der Verschlussaufnahme (38, 138) ausreichend groß ist, um zumindest einen Teil des Verschlusses (36, 136) aufzunehmen, wobei vorzugsweise die Verschlussaufnahme (38, 138) ausreichend groß ist, um den Verschluss (36, 136) vollständig aufzunehmen und besonders bevorzugt die Verschlussaufnahme (38, 138) ein größeres Volumen aufweist als das Volumen des Verschlusses (36, 136).

14. Verfahren zur Herstellung eines Knochenzementteigs (54, 154), insbesondere eines pastenförmigen Polymethylmethacrylat-Knochenzementteigs (54, 154), wobei der Knochenzementteig (54, 154) aus einem Zementpulver (5, 105) und einer Monomerflüssigkeit (4, 104) mit einer Vorrichtung nach einem der vorangehenden Ansprüche hergestellt wird, mit folgenden nacheinander ablaufenden Schritten:
a) die Monomerflüssigkeit (4, 104)wird durch die Verbindung (14, 114) in den Innenraum der Kartusche (1, 101) gedrückt, so dass sich die Monomerflüssigkeit (4, 104) mit dem Zementpulver (5, 105) mischt und dort den Knochenzementteig (54, 154) bildet,
b) der Knochenzementteig (54, 154) wird mit dem Austragskolben (7, 107) in Richtung der Vorderseite der Kartusche (1, 101) gedrückt,
c) der Verschluss (36, 136) wird durch den von dem Knochenzementteig (54, 154) auf den Verschluss (36, 136) wirkenden Druck in die Verschlussaufnahme (38, 138) gedrückt und dabei die Austragsöffnung geöffnet,
d) der Knochenzementteig (54, 154) fließt durch den freien Leitungsquerschnitt (76) durch das Leitungselement (37, 137) und wird aus der Vorrichtung ausgetragen.

15. Verfahren nach Anspruch 14, **dadurch gekennzeichnet, dass**
die Vorrichtung vor Schritt a) in eine Auspressvorrichtung (43, 143) eingesetzt wird, die Auspressvorrichtung (43, 143) aufweisend eine axial vortreibbare Stange (44, 144), wobei vorzugsweise der Austragskolben (7, 107) mit der Stange (44, 144) in Richtung der Austragsöffnung der Kartusche (1, 101) vorgetrieben wird, wobei
nach dem Einsetzen der Vorrichtung in die Auspressvorrichtung (43, 143) ein Förderkolben (6, 106), der innerhalb einer an der Rückseite der Kartusche (1, 101) angeordneten Monomeraufnahme (2, 102) an der Rückseite der Monomeraufnahme (2, 102) beweglich gelagert ist, mit der Stange (44, 144) in Richtung der Kartusche (1, 101) vorgetrieben wird, wobei durch die Bewegung des Förderkolbens (6, 106) ein Monomerflüssigkeitsbehälter (3, 103), in dem die Monomerflüssigkeit (4, 104) enthalten ist, geöffnet wird und die Monomerflüssigkeit (4, 104) aus der Monomeraufnahme (2, 102) in die Kartusche (1, 101) gepresst wird, wobei im Innenraum der Kartusche (1, 101) sich das Zementpulver (5, 105) mit der Monomerflüssigkeit (4, 104) zu dem Knochenzementteig (54, 154) mischt.

16. Verfahren nach einem der Ansprüche 14 oder 15, **dadurch gekennzeichnet, dass**
an der der Austragsöffnung zugewandten Vorderseite des Austragskolbens (7, 107) ein Hohlzylinder (9, 109) angeordnet ist, wobei die Monomerflüssigkeit (4, 104) den Hohlzylinder (9, 109) umfließt, bevor sie bis zur Innenwand der Kartusche (1, 101) gelangt und/oder der Austragskolben (7, 107) auf die Vorderseite der Kartusche (1, 101) trifft, wobei mit dem Hohlzylinder (9, 109) eine weitere Bewegung des Austragskolbens (7, 107) in Richtung der Austragsöffnung blockiert wird und eine Restmenge des Knochenzementteigs (54, 154) in dem von dem Hohlzylinder (9, 109) begrenzten Teil des Innenraums der Kartusche (1, 101) verbleibt, und/oder
in Schritt a) die Monomerflüssigkeit (4, 104) durch zumindest eine für das Zementpulver (5, 105) undurchlässige aber für Gase und die Monomerflüssigkeit (4, 104) durchlässige Verbindung (14, 114) im Austragskolben (7, 107) in die Kartusche (1, 101) gepresst wird, vorzugsweise durch eine Bewegung eines Förderkolbens (6, 106), der mit der Stange der Auspressvorrichtung (43, 143) angetrieben wird, in die Kartusche (1, 101) gepresst wird.

## Claims

1. A device for producing a bone cement paste (54, 154) from a monomer liquid (4, 104) and a cement powder (5, 105) as parent components of the bone cement paste (54, 154) and for delivering the mixed bone cement paste (54, 154), the device having a cartridge (1, 101) with a cylindrical interior, in which the parent components are mixed, the interior of the cartridge (1, 101) being closed at a front side apart from a delivery opening for discharging the bone cement paste (54, 154) from the interior,
a delivery plunger (7, 107), which is arranged in the interior of the cartridge (1, 101) and which is mounted so as to be pushable in the direction of the delivery opening, the cement powder (5, 105), which is arranged in the interior of the cartridge (1, 101) between the delivery opening and the delivery plunger (7, 107),
a closure (36, 136), which closes the delivery opening and which is mounted so as to be movable relative to the delivery opening, and
a line element (37, 137), which is arranged at a front side of the delivery opening, wherein the line element (37, 137) comprises a closure receptacle (38, 138) for receiving at least a part of the closure (36, 136),
wherein the closure (36, 136) is pushable into the closure receptacle (38, 138) by pressure on the bone cement paste (54, 154), in such a way that the delivery opening is opened, wherein, when the closure (36, 136) has been pushed into the closure receptacle (38, 138), the line element (37, 137) provides a free line cross-section (76) through which the bone cement paste (54, 154) is pushable out through the delivery opening and out of the device, wherein
in the delivery plunger (7, 107) at least one connection (14, 114) is provided from the back side of the delivery plunger (7, 107) to the front side of the delivery plunger (7, 107) for introducing the monomer liquid (4, 104) into the interior of the cartridge (1, 101), wherein the at least one connection (14, 114) is permeable to the monomer liquid (4, 104) and gases and impermeable to the cement powder (5, 105).

2. The device according to Claim 1, **characterized in that**
the bone cement paste (54, 154) flows around the closure (36, 136) in the closure receptacle (38, 138) when the bone cement paste (54, 154) flows through the line element (37, 137), preferably the bone cement paste (54, 154) flows along at least one side face or circumferential surface of the closure (36, 136) past said closure (36, 136).

3. The device according to any one of the preceding claims, **characterized in that**
the closure (36, 136) becomes firmly lodged in the closure receptacle (38, 138) when it has been pushed out of the delivery opening into the closure receptacle (38, 138) and/or
the free line cross-section (76) is delimited on one side at least in areas by the closure (36, 136), preferably by a side face or a circumferential surface of the closure (36, 136).

4. The device according to any one of the preceding claims, **characterized in that**
the closure (36, 136) is cylindrical at least in parts, in particular is completely cylindrical, and the closure receptacle (38, 138) forms a hollow-cylindrical sleeve, wherein at least one channel is provided in the circumferential surface of the hollow-cylindrical sleeve, wherein the at least one channel provides the free line cross-section (76), wherein preferably
the internal diameter of the hollow-cylindrical sleeve is greater than the external diameter of the closure (36, 136), preferably is at least 1 mm greater than the external diameter of the closure (36, 136), particularly preferably is between 1 mm and 10 mm greater than the external diameter of the closure (36, 136), and/or
the axial length of the interior of the hollow-cylindrical sleeve is greater than the axial length of the closure (36, 136), preferably is at least 1 mm greater than the axial length of the closure (36, 136), particularly preferably is between 1 mm and 20 mm greater than the axial length of the closure (36, 136).

5. The device according to any one of the preceding claims, **characterized in that**
spacers (39, 40, 139, 140) are provided in the closure receptacle (38, 138) for spacing the closure (36, 136) from the internal wall of the closure receptacle (38, 138), wherein the spacers are preferably bars (39, 40, 139, 140) which are particularly preferably oriented in the direction of movement of the closure (36, 136) and/or are oriented in the direction of flow of the bone cement paste (54, 154).

6. The device according to any one of the preceding claims, **characterized in that**
the length of the closure (36, 136) is greater in its direction of movement than the width in the directions perpendicular thereto and/or
the free line cross-section (76) is at least half as large as the cross-section of the delivery opening, preferably at least as large as the cross-section of the delivery opening.

7. The device according to any one of the preceding claims, **characterized in that**
a limit stop (40, 140) for limiting movement of the closure (36, 136) is arranged in the closure receptacle (38, 138) at a front end wall of the closure receptacle (38, 138) which is remote from the delivery opening, wherein the limit stop (40, 140) spaces the closure (36, 136), when fully pushed in, from the front end wall at the front side of the closure receptacle (38, 138), such that the free line cross-section (76) remains between the front side of the closure (36, 136) and the front end wall.

8. The device according to any one of the preceding claims, **characterized in that**
the device having a monomer receptacle (2, 102) in which the monomer liquid (4, 104), in particular a monomer liquid container (3, 103) containing the monomer liquid (4, 104), is contained, wherein a back side of the cartridge (1, 101) is connected with a front side of the monomer receptacle (2, 102), preferably connected in such a way that the interior of the cartridge (1, 101) is aligned with the interior of the monomer receptacle (2, 102), wherein
an interior of the monomer receptacle (2, 102) and the interior of the cartridge (1, 101) are connected together via a connection (14, 114) which is permeable to the monomer liquid (4, 104) and gases but impermeable to the cement powder (5, 105) and/or
the monomer receptacle (2, 102) has a cylindrical interior in which the monomer liquid (4, 104), in particular a monomer liquid container (3, 103) containing the monomer liquid (4, 104), is arranged.

9. The device according to Claim 8, **characterized in that**
at least one ventilation opening (20, 120), which connects the interior of the monomer receptacle (2, 102) with the surrounding environment, is arranged in the wall of the monomer receptacle (2, 102), and/or
a conveying plunger (6, 106) movable in the longitudinal direction of the receptacle (2, 102) is arranged in the monomer receptacle (2, 102), which conveying plunger is advanceable from a back side of the monomer receptacle (2, 102) in the direction of the front side, wherein the monomer liquid (4, 104), in particular a monomer liquid container (3, 103) containing the monomer liquid (4, 104), is arranged between the conveying plunger (6, 106) and the delivery plunger (7, 107).

10. The device according to any one of the preceding claims, **characterized in that**
a fastening means (8, 108) is arranged on a back side of the device for fastening an expulsion device (43, 143) with which the delivery plunger (7, 107) is pushable in the direction of the delivery opening.

11. The device according to any one of the preceding claims, **characterized in that**
a hollow cylinder (9, 109) is arranged at a front side of the delivery plunger (7, 107) facing the delivery opening, wherein the hollow cylinder (9, 109) is open at its front side facing the delivery opening and preferably extends from the front side of the delivery plunger (7, 107) at least 3 mm into the interior of the cartridge (1, 101), wherein
the hollow cylinder (9, 109) blocks further movement of the delivery plunger (7, 107) in the direction of the front side of the cartridge (1, 101) when the front side of the hollow cylinder (9, 109) rests against the front side of the interior of the cartridge (1, 101), such that the delivery plunger (7, 107) is spaced from the front side of the interior of the cartridge (1, 101) and a dead volume remains in the interior of the cartridge (1, 101).

12. The device according to any one of the preceding claims, **characterized in that**
the at least one connection (14, 114) leads from the delivery plunger (7, 107) inside the hollow cylinder (9, 109) or through lines in the hollow cylinder (9, 109) at the front side of the hollow cylinder (9, 109) into the interior of the cartridge (1, 101), and
the closure (36, 136) has an indentation at the back side facing the interior of the cartridge (1, 101), in which indentation a frontmost part of the cement powder (5, 105) is contained.

13. The device according to any one of the preceding claims, **characterized in that**
the volume of the closure receptacle (38, 138) is sufficiently large to accommodate at least a part of the closure (36, 136), wherein the closure receptacle (38, 138) is preferably sufficiently large to accommodate the closure (36, 136) completely and particularly preferably the closure receptacle (38, 138) has a larger volume than the volume of the closure (36, 136).

14. A method for producing a bone cement paste (54, 154), in particular a polymethyl methacrylate bone cement paste (54, 154), wherein the bone cement paste (54, 154) is produced from a cement powder (5, 105) and a monomer liquid (4, 104) using a device according to any one of the preceding claims, with the following succession of steps:
a) the monomer liquid (4, 104) is pushed into the interior of the cartridge (1, 101), such that the monomer liquid (4, 104) mixes with the cement powder (5, 105) and there forms the bone cement paste (54, 154),
b) the bone cement paste (54, 154) is pushed with the delivery plunger (7, 107) in the direction of a front side of the cartridge (1, 101),
c) the closure (36, 136) is pushed into the closure receptacle (38, 138) by the pressure of the bone cement paste (54, 154) acting on the closure (36, 136) and the delivery opening is opened in the process,
d) the bone cement paste (54, 154) flows through the line element (37, 137) through the free line cross-section (76) and is delivered from the device.

15. The method according to Claim 14, **characterized in that**
the device is inserted into an expulsion device (43, 143) prior to step a), the expulsion device (43, 143) having an axially advanceable rod (44, 144), wherein the delivery plunger (7, 107) is preferably advanced with the rod (44, 144) in the direction of the delivery opening of the cartridge (1, 101), wherein
after insertion of the device into the expulsion device (43, 143), a conveying plunger (6, 106), which is mounted movably inside a monomer receptacle (2, 102) arranged on the back side of the cartridge (1, 101) at the back side of the monomer receptacle (2, 102), is advanced with the rod (44, 144) in the direction of the cartridge (1, 101), wherein through movement of the conveying plunger (6, 106) a monomer liquid container (3, 103), in which the monomer liquid (4, 104) is contained, is opened and the monomer liquid (4, 104) is pressed out of the monomer receptacle (2, 102) into the cartridge (1, 101), wherein the cement powder (5, 105) mixes with the monomer liquid (4, 104) in the interior of the cartridge (1, 101) to yield the bone cement paste (54, 154).

16. The method according to any one of Claims 14 or 15, **characterized in that**
a hollow cylinder (9, 109) is arranged at a front side of the delivery plunger (7, 107) facing the delivery opening, wherein the monomer liquid (4, 104) flows around the hollow cylinder (9, 109) before arriving at the internal wall of the cartridge (1, 101) and/or the delivery plunger (7, 107) meets with a front side of the cartridge (1, 101), wherein further movement of the delivery plunger (7, 107) in the direction of the delivery opening is blocked with the hollow cylinder (9, 109) and a residual quantity of the bone cement paste (54, 154) remains in the part of the interior of the cartridge (1, 101) delimited by the hollow cylinder (9, 109), and/or
in step a) the monomer liquid (4, 104) is pressed through at least one connection (14, 114) in the delivery plunger (7, 107) impermeable to the cement powder (5, 105) but permeable to gases and the monomer liquid (4, 104) into the cartridge (1, 101), preferably is pressed into the cartridge (1, 101) by movement of a conveying plunger (6, 106) which is driven with the rod of the expulsion device (43, 143).

## Revendications

1. Dispositif pour la fabrication d'une pâte de ciment osseux (54, 154) à partir d'un liquide monomère (4, 104) et d'une poudre de ciment (5, 105) comme composants de départ de la pâte de ciment osseux (54, 154) et pour le déversement de la pâte de ciment osseux (54, 154) mélangée, le dispositif présentant
une cartouche (1, 101) avec un espace intérieur cylindrique, dans lequel les composants de départ sont mélangés, l'espace intérieur de la cartouche (1, 101) étant fermé sur la face avant, à l'exception d'un orifice de sortie pour l'éjection de la pâte de ciment osseux (54, 154) de l'espace intérieur,
un piston de décharge (7, 107) qui est disposé dans l'espace intérieur de la cartouche (1, 101) et qui peut être enfoncé en direction de l'orifice de sortie, la poudre de ciment (5, 105) qui est placée dans l'espace intérieur de la cartouche (1, 101) entre l'orifice de sortie et le piston de décharge (7, 707), un bouchon (36, 136) qui obture l'orifice de sortie et qui est monté de façon mobile contre l'orifice de sortie et
un élément de conduite (37, 137) qui est disposé sur la face avant de l'orifice de sortie, l'élément de conduite (37, 137) comprenant une fixation du bouchon (38, 138) pour la réception au moins d'une partie du bouchon (36, 136), le bouchon (36, 136) pouvant être enfoncé dans la fixation du bouchon (38, 138) par une pression sur la pâte de ciment osseux (54, 154), de telle sorte que l'orifice de sortie s'ouvre, l'élément de conduite (37, 137) fournissant, avec le bouchon (36, 136) enfoncé dans la fixation du bouchon (38, 138), une section transversale libre de la conduite (76) par laquelle la pâte de ciment osseux (54, 154) peut être pressée de l'orifice de sortie et du dispositif, au moins une liaison (14, 114) de la face arrière du piston de décharge (7, 107) à la face avant du piston de décharge (7, 107) étant prévue dans le piston de décharge (7, 107) pour l'introduction du liquide monomère (4, 104) dans l'espace intérieur de la cartouche (1, 101), au moins une liaison (14, 114) étant perméable au liquide monomère (4, 104) et aux gaz et imperméable à la poudre de ciment (5, 105).

2. Dispositif conformément à la revendication 1, **caractérisé en ce que** la pâte de ciment osseux (54, 154) entoure le bouchon (36, 136) dans la fixation du bouchon (38, 138) lorsque la pâte de ciment osseux (54, 154) coule par l'élément de conduite (37, 137), préférablement la pâte de ciment osseux (54, 154) coule devant le bouchon (36, 136) le long au moins d'une surface latérale ou surface enveloppante du bouchon (36, 136).

3. Dispositif conformément à l'une des revendications précédentes, **caractérisé en ce que**
le bouchon (36, 136) est fixé dans la fixation du bouchon (38, 138) lorsqu'il est enfoncé dans la fixation du bouchon (38, 138) depuis l'orifice de sortie et/ou la section transversale libre de la conduite (76) est limitée, au moins localement sur un côté, par le bouchon (36, 136), préférablement par une surface latérale ou une surface enveloppante du bouchon (36, 136).

4. Dispositif conformément à l'une des revendications précédentes, **caractérisé en ce que**,
le bouchon (36, 136) est au moins localement cylindrique, est en particulier entièrement cylindrique, et que la fixation du bouchon (38, 138) constitue une gaine en forme de cylindre creux, au moins un conduit étant prévu dans la surface enveloppante de la gaine en forme de cylindre creux, au moins un conduit fournissant la section transversale libre de la conduite (76), préférablement
le diamètre intérieur de la gaine en forme de cylindre creux étant plus grand que le diamètre extérieur du bouchon (36, 136), préférablement au moins 1 mm plus grand que le diamètre extérieur du bouchon (36, 136), plus préférablement entre 1 mm et 10 mm plus grand que le diamètre extérieur du bouchon (36, 136) et/ou
la longueur axiale de l'espace intérieur de la gaine en forme de cylindre creux étant plus grande que la longueur axiale du bouchon (36, 136), préférablement au moins 1 mm plus grande que la longueur axiale du bouchon (36, 136), plus préférablement entre 1 mm et 20 mm plus grande que la longueur axiale du bouchon (36, 136).

5. Dispositif conformément à l'une des revendications précédentes, **caractérisé en ce que**
des entretoises (39, 40, 139, 140) pour l'espacement du bouchon (36, 136) par rapport à la paroi intérieure de la fixation du bouchon (38, 138) sont prévues dans la fixation du bouchon (38, 138), les entretoises étant préférablement des tasseaux (39, 40, 139, 140) qui sont plus préférablement alignés dans le sens de mouvement du bouchon (36, 136) et/ou qui sont alignés dans le sens d'écoulement de la pâte de ciment osseux (54, 154).

6. Dispositif conformément à l'une des revendications précédentes, **caractérisé en ce que**
la longueur du bouchon (36, 136) est plus grande dans son sens de mouvement que la largeur dans les sens perpendiculaires à celle-ci et/ou la section transversale libre de la conduite (76) est au moins la moitié de la section transversale de l'orifice de sortie, préférablement au moins aussi grande que la section transversale de l'orifice de sortie.

7. Dispositif conformément à l'une des revendications précédentes, **caractérisé en ce que**
une butée (40, 140) pour la limitation du mouvement du bouchon (36, 136) est disposée sur la paroi frontale, détournée de l'orifice de sortie, dans la fixation du bouchon (38, 138), la butée (40, 140) espaçant le bouchon (36, 136) à l'état entièrement enfoncé de la paroi frontale sur la face avant de la fixation du bouchon (38, 138), de telle sorte que la section transversale libre de la conduite (76) reste entre la face avant du bouchon (36, 136) et la paroi frontale.

8. Dispositif conformément à l'une des revendications précédentes, **caractérisé en ce que**
le dispositif présente un logement de monomère (2, 102) dans lequel le liquide monomère (4, 104) est contenu, en particulier un réservoir de liquide monomère (3, 103) contenant le liquide monomère (4, 104), la face arrière de la cartouche (1, 101) étant reliée à la face avant du logement de monomère (2, 102), préférablement reliée de telle sorte que l'espace intérieur de la cartouche (1, 101) soit aligné avec l'espace intérieur du logement de monomère (2, 102),
un espace intérieur du logement de monomère (2, 102) et l'espace intérieur de la cartouche (1, 101) étant reliés par une liaison (14, 114) perméable au liquide monomère (4, 104) et aux gaz, mais imperméable à la poudre de ciment (5, 105) et/ou
le logement de monomère (2, 102) présentant un espace intérieur cylindrique dans lequel le liquide monomère (4, 104) est placé, en particulier un réservoir de liquide monomère (3, 103) contenant le liquide monomère (4, 104).

9. Dispositif conformément à la revendication 8, **caractérisé en ce que**
au moins un orifice de ventilation (20, 120) qui relie l'espace intérieur du logement de monomère (2, 102) à l'environnement, est disposé dans la paroi du logement de monomère (2, 102) et/ou
un piston d'alimentation (6, 106) mobile dans le sens longitudinal du logement de monomère (2, 102), lequel peut être actionné depuis une face arrière du logement de monomère (2, 102) en direction de la face avant, est disposé dans le logement de monomère (2, 102), le liquide monomère (4, 104), en particulier un réservoir de liquide monomère (3, 103) contenant le liquide monomère (4, 104), étant disposé entre le piston d'alimentation (6, 106) et le piston de décharge (7, 107).

10. Dispositif conformément à l'une des revendications précédentes, **caractérisé en ce que**
un moyen de fixation (8, 108) pour la fixation d'un dispositif de pressage (43, 143) permettant d'enfoncer le piston de décharge (7, 107) en direction de l'orifice de sortie, est disposé sur la face arrière du dispositif.

11. Dispositif conformément à l'une des revendications précédentes, **caractérisé en ce que**
un cylindre creux (9, 109) est disposé sur la face avant du piston de décharge (7, 107), tournée vers l'orifice de sortie, le cylindre creux (9, 109) étant ouvert sur sa face avant, tournée vers l'orifice de sortie, et s'étendant préférablement depuis la face avant du piston de décharge (7, 107) au moins de 3 mm dans l'espace intérieur de la cartouche (1, 101),
le cylindre creux (9, 109) bloquant un mouvement supplémentaire du piston de décharge (7, 107) en direction de la face avant de la cartouche (1, 101) lorsque la face avant du cylindre creux (9, 109) est adjacente à la face avant de l'espace intérieur de la cartouche (1, 101), de telle sorte que le piston de décharge (7, 107) soit espacé de la face avant de l'espace intérieur de la cartouche (1, 101) et qu'un volume mort reste dans l'espace intérieur de la cartouche (1, 101).

12. Dispositif conformément à l'une des revendications précédentes, **caractérisé en ce que**
au moins une liaison (14, 114) débouche dans l'espace intérieur de la cartouche (1, 101) depuis le piston de décharge (7, 107) à l'intérieur du cylindre creux (9, 109) ou par des conduites dans le cylindre creux (9, 109) sur la face avant du cylindre creux (9, 109) et que
le bouchon (36, 136) présente, sur la face arrière tournée vers l'espace intérieur de la cartouche (1, 101), un creux dans lequel la partie la plus avancée de la poudre de ciment (5, 105) est contenue.

13. Dispositif conformément à l'une des revendications précédentes, **caractérisé en ce que**
le volume de la fixation du bouchon (38, 138) est suffisamment grand pour recevoir au moins une partie du bouchon (36, 136), le bouchon (36, 136) étant préférablement suffisamment grand pour recevoir entièrement le bouchon (36, 136) et plus préférablement la fixation du bouchon (38, 138) présentant un plus grand volume que le volume du bouchon (36, 136).

14. Procédé pour la fabrication d'une pâte de ciment osseux (54, 154), en particulier d'une pâte de ciment osseux de méthacrylate de polyméthyle (54, 154) à l'état pâteux, la pâte de ciment osseux (54, 154) étant fabriquée à partir d'une poudre de ciment (5, 105) et d'un liquide monomère (4, 104) au moyen d'un dispositif conformément à l'une des revendications précédentes, avec les étapes suivantes se déroulant successivement :
a) le liquide monomère (4, 104) est pressé dans l'espace intérieur de la cartouche (1, 101) par la liaison (14, 114) de façon à se mélanger avec la poudre de ciment (5, 105) et à y former la pâte de ciment osseux (54, 154),
b) la pâte de ciment osseux (54, 154) est pressée avec le piston de décharge (7, 707) en direction de la face avant de la cartouche (1, 101),
c) le bouchon (36, 136) est enfoncé dans la fixation du bouchon (38, 138) par la pression qui agit de la pâte de ciment osseux (54, 154) sur le bouchon (36, 136), ce qui ouvre l'orifice de sortie,
d) la pâte de ciment osseux (54, 154) coule par la section transversale libre de la conduite (76) par l'élément de conduite (37, 137) et est déversée du dispositif,

15. Procédé conformément à la revendication 14, **caractérisé en ce que**
le dispositif est inséré dans un dispositif de pressage (43, 143) avant l'étape a), le dispositif de pressage (43, 143) présentant une tige (44, 144) pouvant être actionnée axialement, préférablement le piston de décharge (7, 107) étant actionné avec la tige (44, 144) en direction de l'orifice de sortie de la cartouche (1, 101),
après l'insertion du dispositif dans le dispositif de pressage (43, 143), un piston d'alimentation (6, 106) qui est monté de façon mobile sur la face arrière du logement de monomère (2, 102) à l'intérieur d'un logement de monomère (2, 102) disposé sur la face arrière de la cartouche (1, 101), étant actionné avec la tige (44, 144) en direction de la cartouche (1, 101), et par le mouvement du piston d'alimentation (6, 106), un réservoir de liquide monomère (3, 103) dans lequel le liquide monomère (4, 104) est contenu, étant ouvert et le liquide monomère (4, 104) étant pressé du logement de monomère (2, 102) dans la cartouche (1, 101), la poudre de ciment (5, 105) se mélangeant avec le liquide monomère (4, 104) pour former la pâte de ciment osseux (54, 154) dans l'espace intérieur de la cartouche (1, 101).

16. Procédé conformément à l'une des revendications 14 ou 15, **caractérisé en ce que**
un cylindre creux (9, 109) est disposé sur la face avant du piston de décharge (7, 707), tourné vers l'orifice de sortie, le liquide monomère (4,104) entourant le cylindre creux (9, 109) avant d'atteindre la paroi intérieure de la cartouche (1, 101) et/ou le piston de décharge (7, 107) entrant en contact avec la face avant de la cartouche (1, 101), un mouvement supplémentaire du piston de décharge (7, 107) en direction de l'orifice de sortie étant bloqué avec le cylindre creux (9, 109) et une quantité restante de pâte de ciment osseux (54, 154) restant dans la partie de l'espace intérieur de la cartouche (1, 101), limitée par le cylindre creux (9, 109), et/ou
dans l'étape a), le liquide monomère (4,104) est pressé dans la cartouche (1, 101) par au moins une liaison (14, 114) imperméable à la poudre de ciment (5, 105), mais perméable aux gaz et au liquide monomère (4, 104) dans le piston de décharge (7, 107), est pressé dans la cartouche (1, 101) préférablement par un mouvement du piston d'alimentation (6, 106) qui est actionné avec la tige du dispositif de pressage (43, 143).
